(19) Europäisches Patentamt
European Patent Office
Office européen des brevets

(11) EP 4 382 123 A1

(12) # EUROPEAN PATENT APPLICATION
published in accordance with Art. 153(4) EPC

(43) Date of publication:
**12.06.2024 Bulletin 2024/24**

(21) Application number: **22852342.9**

(22) Date of filing: **05.08.2022**

(51) International Patent Classification (IPC):
***A61K 39/395*** (2006.01) ***A61K 31/4709*** (2006.01)
***A61P 35/00*** (2006.01)

(52) Cooperative Patent Classification (CPC):
**A61K 31/4709; A61K 39/395; A61P 35/00**

(86) International application number:
**PCT/CN2022/110539**

(87) International publication number:
**WO 2023/011631 (09.02.2023 Gazette 2023/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.08.2021 CN 202110897167**

(71) Applicant: **CHIA TAI TIANQING PHARMACEUTICAL GROUP CO., LTD.**
**Lianyungang,**
**Jiangsu 222062 (CN)**

(72) Inventors:
- **ZHANG, Xiquan**
  **Nanjing City, Jiangsu 211100 (CN)**
- **WANG, Xunqiang**
  **Nanjing City, Jiangsu 211100 (CN)**
- **YU, Ding**
  **Nanjing City, Jiangsu 211100 (CN)**

(74) Representative: **v. Bezold & Partner Patentanwälte - PartG mbB**
**Ridlerstraße 57**
**80339 München (DE)**

Remarks:
The complete document including Reference Table(s) and the Sequence Listing(s) can be downloaded from the EPO website

(54) **PHARMACEUTICAL COMPOSITION FOR TREATING SMALL CELL LUNG CANCER**

(57) Provided is a pharmaceutical composition for treating small cell lung cancer, which comprises an anti-PD-L1 antibody, anlotinib or a pharmaceutically acceptable salt thereof, and a chemotherapeutic drug. Further provided is the use of the pharmaceutical composition or a kit containing the anti-PD-L1 antibody, anlotinib or a pharmaceutically acceptable salt thereof and the chemotherapeutic drug in the preparation of a drug for treating small cell lung cancer. Further provided is a method for treating small cell lung cancer, comprising administering to a patient in need thereof a therapeutically effective amount of the anti-PD-L1 antibody, anlotinib or a pharmaceutically acceptable salt thereof, and the chemotherapeutic drug.

EP 4 382 123 A1

## Description

### TECHNICAL FIELD

The present application relates to the field of biopharmaceuticals, and particularly, to a pharmaceutical combination for treating small cell lung cancer, a kit, and use thereof.

### BACKGROUND

Tyrosine kinase is a group of enzymes that catalyze the phosphorylation of tyrosine residues in proteins. It plays an important role in intracellular signal transduction, takes part in the regulation, signaling and development of normal cells, and is closely related to the proliferation, differentiation, migration and apoptosis of tumor cells. Many receptor tyrosine kinases are related to tumorigenesis and can be classified into epidermal growth factor receptor (EGFR), platelet-derived growth factor receptor (PDGFR), vascular endothelial growth factor receptor (VEGFR), fibroblast growth factor receptor (FGFR) and the like according to the structure of the extracellular domain.

PD-L1 (programmed death-ligand 1), also known as CD247 or B7-H1, is a ligand for programmed cell death protein 1 (PD-1). PD-L1 is highly expressed on the surface of various tumor cells, and the malignant degree and poor prognosis of tumors are closely related to the expression level of PD-L1. In a tumor microenvironment, PD-L1 on cancer cell surface inhibits the activation and proliferation of T cells, induces effector T cell exhaustion or anergy, promotes the apoptosis of T cells, and stimulates the differentiation of helper T cells into regulatory T cells by binding to PD-1 or CD80 on T cell surface, thus preventing the killing effect of T cells on tumor cells. Anti-PD-L1 antibodies can prevent the related negative regulation signals from being initiated and transducted by blocking the interaction of PD-L1 with PD-1 and CD80, thereby avoiding the inhibited activity of effector T cells in tumor microenvironment and enabling T cells to exert the functions of killing and inhibiting tumor cells. Anti-PD-L1 antibodies can directly act on tumor tissues, thus featuring high specificity and safety.

Small cell lung cancer (SCLC) is the most malignant type of lung cancer, which features rapid progression, early metastasis, easy relapse, and the like. It accounts for about 15-20% of new lung cancer cases and is closely related to long-term smoking. SCLC is highly invasive and usually associated with poor prognosis, with the 5-year survival rate being less than 5% and the mean survival time of untreated patients being only 2-4 months. SCLC is more sensitive to chemotherapy and radiation therapy compared to other types of lung cancer. The combination treatment with etoposide and platinum-based anti-tumor drugs is the standard first-line treatment for treating extensive-stage SCLC. However, most treated patients will experience disease progression within 3 months along with a high recurrence rate and drug resistance rate. Therefore, there are still many challenges in the treatment.

WO2016022630 discloses a group of anti-PD-L1 antibodies, which have a higher affinity to PD-L1 and can significantly inhibit the interaction between PD-L1 and PD-1 on the cell surface and promote T cells to secrete IL-2 and INF-$\gamma$.

Although patients with proliferative diseases (for example, cancers) have many treatment options, there's still a need for more effective therapeutic agents for clinical use, in particular the combined use of more than one drug.

### SUMMARY

In one aspect, the present application provides a pharmaceutical combination for use in treating small cell lung cancer, comprising an anti-PD-L1 antibody and anlotinib or a pharmaceutically acceptable salt thereof, and further comprising a chemotherapeutic drug.

Further, the anti-PD-L1 antibody comprises the following amino acid sequences: a heavy chain CDR1 region having at least 80% homology to the amino acid sequence set forth in SEQ ID NO: 1 or SEQ ID NO: 4; a heavy chain CDR2 region having at least 80% homology to the amino acid sequence set forth in SEQ ID NO: 2 or SEQ ID NO: 5; a heavy chain CDR3 region having at least 80% homology to the amino acid sequence set forth in SEQ ID NO: 3 or SEQ ID NO: 6; a light chain CDR1 region having at least 80% homology to the amino acid sequence set forth in SEQ ID NO: 7 or SEQ ID NO: 10; a light chain CDR2 region having at least 80% homology to the amino acid sequence set forth in SEQ ID NO: 8 or SEQ ID NO: 11; and a light chain CDR3 region having at least 80% homology to the amino acid sequence set forth in SEQ ID NO: 9 or SEQ ID NO: 12. Still further, the anti-PD-L1 antibody comprises the following amino acid sequences: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12. Still further, the anti-PD-L1 antibody comprises: a heavy chain CDR1 region

having the amino acid sequence set forth in SEQ ID NO: 1; a heavy chain CDR2 region having the amino acid sequence set forth in SEQ ID NO: 2; a heavy chain CDR3 region having the amino acid sequence set forth in SEQ ID NO: 3; a light chain CDR1 region having the amino acid sequence set forth in SEQ ID NO: 7; a light chain CDR2 region having the amino acid sequence set forth in SEQ ID NO: 8; and a light chain CDR3 region having the amino acid sequence set forth in SEQ ID NO: 9. Still further, the anti-PD-L1 antibody comprises the following amino acid sequences: a heavy chain variable region having at least 80% homology to the amino acid sequence set forth in SEQ ID NO: 13 or SEQ ID NO: 14; and a light chain variable region having at least 80% homology to the amino acid sequence set forth in SEQ ID NO: 15 or SEQ ID NO: 16. Still further, the anti-PD-L1 antibody comprises: a heavy chain variable region of humanized antibodies selected from the group consisting of hu13C5-hIgG1, hu13C5-hIgG4, hu5G11-hIgG1, and hu5G11-hIgG4; and a light chain variable region of humanized antibodies selected from the group consisting of hu13C5-hIgG1, hu13C5-hIgG4, hu5G11-hIgG1, and hu5G11-hIgG4.

Still further, the chemotherapeutic drug is one or more selected from the group consisting of a platinum-based anti-tumor drug and a topoisomerase inhibitor. In some embodiments, the platinum-based anti-tumor drug includes but is not limited to one or more of cisplatin, carboplatin, nedaplatin, dicycloplatin, picoplatin, oxaliplatin, miriplatin, or lobaplatin. In some specific embodiments, the platinum-based anti-tumor drug is one or more selected from the group consisting of carboplatin, cisplatin, nedaplatin, picoplatin, miriplatin, and lobaplatin. In some specific embodiments, the platinum-based anti-tumor drug is selected from carboplatin. In some specific embodiments, the platinum-based anti-tumor drug is selected from cisplatin. In some embodiments, the topoisomerase inhibitor includes but is not limited to a topoisomerase I inhibitor, a topoisomerase II inhibitor, and a topoisomerase I/II dual inhibitor; in some embodiments, the topoisomerase inhibitor is selected from a topoisomerase II inhibitor. In some embodiments, the topoisomerase inhibitor includes but is not limited to one or more of camptothecin, topotecan, adriamycin, daunorubicin, epirubicin, idarubicin, mitoxantrone, irinotecan, etoposide, or teniposide. In some embodiments, the topoisomerase inhibitor is one or more selected from the group consisting of topotecan, adriamycin, epirubicin, mitoxantrone, irinotecan, and etoposide. In some specific embodiments, the topoisomerase inhibitor is selected from the group consisting of etoposide. In some embodiments, the chemotherapeutic drug is selected from the group consisting of a platinum-based anti-tumor drug and etoposide. In some embodiments, the chemotherapeutic drug is selected from the group consisting of carboplatin and a topoisomerase inhibitor. In some embodiments, the chemotherapeutic drug comprises one or more selected from the group consisting of carboplatin, cisplatin, nedaplatin, picoplatin, miriplatin and lobaplatin, and one or more selected from the group consisting of topotecan, adriamycin, epirubicin, mitoxantrone, irinotecan and etoposide. In some embodiments, the chemotherapeutic drug is selected from the group consisting of carboplatin and etoposide.

In some embodiments, the pharmaceutical combination further comprises a pharmaceutically acceptable carrier.

In another aspect, the present application provides a pharmaceutical composition for use in treating small cell lung cancer, comprising a first pharmaceutical combination, and optionally, a second pharmaceutical combination. In some embodiments, the pharmaceutical combination comprises a first pharmaceutical combination administered to a patient in need in a first treatment phase, and optionally, a second pharmaceutical combination administered to the patient in need in a second treatment phase. In some embodiments, the first treatment phase comprises 1 to 10 treatment cycles, preferably 2 to 8 treatment cycles, and most preferably 4 treatment cycles.

In some embodiments, the first pharmaceutical combination comprises an anti-PD-L1 antibody, anlotinib or a pharmaceutically acceptable salt thereof, and a chemotherapeutic drug.

In some embodiments, the second pharmaceutical combination comprises the anti-PD-L1 antibody and anlotinib or the pharmaceutically acceptable salt thereof.

In one specific embodiment, the pharmaceutical combination comprises a first pharmaceutical combination administered to a patient in need in the first treatment phase, and optionally, a second pharmaceutical combination administered to a patient in need in the second treatment phase, wherein the first pharmaceutical combination comprises an anti-PD-L1 antibody, anlotinib or a pharmaceutically acceptable salt thereof, and a chemotherapeutic drug, and the second pharmaceutical combination comprises an anti-PD-L1 antibody and anlotinib or a pharmaceutically acceptable salt thereof. In some embodiments, the first treatment phase comprises 1 to 10 treatment cycles, preferably 2 to 8 treatment cycles, and most preferably 4 treatment cycles.

In some embodiments, the pharmaceutical combination further comprises a pharmaceutically acceptable carrier.

In some embodiments, in the pharmaceutical combination described herein comprising the anti-PD-L1 antibody, anlotinib or the pharmaceutically acceptable salt thereof, and the chemotherapeutic drug, the anti-PD-L1 antibody, anlotinib or the pharmaceutically acceptable salt thereof and the chemotherapeutic drug are each present in the form of a pharmaceutical composition and can be administered simultaneously, non-simultaneously, or sequentially. In some embodiments, the pharmaceutical combination comprises a pharmaceutical composition of the anti-PD-L1 antibody, a pharmaceutical composition of anlotinib or the pharmaceutically acceptable salt thereof, and a pharmaceutical composition of the chemotherapeutic drug.

Further, the pharmaceutical combination disclosed herein is packaged in a kit further comprising an instruction for the treatment of small cell lung cancer.

In some embodiments, the present application provides a kit for use in treating small cell lung cancer, comprising the anti-PD-L1 antibody, anlotinib or the pharmaceutically acceptable salt thereof, and the chemotherapeutic drug. In some embodiments, the anti-PD-L1 antibody is contained in a first compartment, anlotinib or the pharmaceutically acceptable salt thereof is contained in a second compartment, and the chemotherapeutic drug is contained in an additional compartment. Optionally, the number of compartments in the kit can be increased as appropriate depending on the number of chemotherapeutic drugs. The components can be administered to a patient in need simultaneously, non-simultaneously, or sequentially. In some embodiments, the kit further comprises an instruction for the combined use of the anti-PD-L1 antibody, anlotinib or the pharmaceutically acceptable salt thereof, and the chemotherapeutic drug for treating small cell lung cancer. In some embodiments, the kit comprises a pharmaceutical composition of the anti-PD-L1 antibody, a pharmaceutical composition of anlotinib or the pharmaceutically acceptable salt thereof, and a pharmaceutical composition of the chemotherapeutic drug.

Further, the kit is suitable for administration within a single treatment cycle (e.g., a treatment cycle of 21 days), and comprises a pharmaceutical composition comprising 600-2400 mg of the anti-PD-L1 antibody and a pharmaceutical composition comprising 84-168 mg of anlotinib. In some embodiments, the treatment cycle comprises 1 week, 2 weeks, 3 weeks, or 4 weeks.

The amount of the anti-PD-L1 antibody administered can be determined according to the severity of the disease, the response of the disease, any treatment-related toxicity, and the age and health of the patient. For example, the daily dose of the anti-PD-L1 antibody administered may be 600-2400 mg. In some embodiments, the daily dose of the anti-PD-L1 antibody administered may be 600 mg, 800 mg, 1000 mg, 1200 mg, 1400 mg, 1600 mg, 1800 mg, 2000 mg, 2200 mg, or 2400 mg. In some embodiments, the anti-PD-L1 antibody is administered parenterally. In some embodiments, the anti-PD-L1 antibody is administered intravenously. In some embodiments, the pharmaceutical composition of the anti-PD-L1 antibody has a concentration of 10-60 mg/mL. In some embodiments, the pharmaceutical composition of the anti-PD-L1 antibody has a concentration of 10 mg/mL, 20 mg/mL, 30 mg/mL, 40 mg/mL, 50 mg/mL, or 60 mg/mL.

The administration regimen for the anti-PD-L1 antibody can be determined comprehensively depending on the activity and toxicity of the drug, the tolerance of a patient, and the like. In some embodiments, for the anti-PD-L1 antibody, the treatment cycle comprises 1 week, 2 weeks, 3 weeks, or 4 weeks. In some embodiments, the anti-PD-L1 antibody is administered once every week, every 2 weeks, every 3 weeks, or every 4 weeks. In some embodiments, the anti-PD-L1 antibody is administered at a dose of 600-2400 mg per treatment cycle. In some specific embodiments, the anti-PD-L1 antibody is administered at a dose of 1200 mg per treatment cycle. In some embodiments, the anti-PD-L1 antibody is administered at a dose of 600-2400 mg once every 3 weeks. In some embodiments, anlotinib or the pharmaceutically acceptable salt thereof is administered at a daily dose of 6 mg, 8 mg, 10 mg, or 12 mg on an administration regimen of consecutively 2-week treatment and then 1-week interruption.

In some embodiments, the pharmaceutical combination described herein comprising the anti-PD-L1 antibody, anlotinib or the pharmaceutically acceptable salt thereof, and the chemotherapeutic drug comprises a pharmaceutical composition of the anti-PD-L1 antibody and a pharmaceutical composition of anlotinib, wherein the pharmaceutical composition of the anti-PD-L1 antibody is prepared in a unit dose or in multiple doses suitable for administering to a patient 600-2400 mg of the anti-PD-L1 antibody at a first administration, and the pharmaceutical composition of anlotinib or the pharmaceutically acceptable salt thereof is prepared in a unit dose suitable for administering to the patient 6 mg, 8 mg, 10 mg, and/or 12 mg of anlotinib or the pharmaceutically acceptable salt thereof daily for 14 consecutive days.

In some embodiments, the pharmaceutical combination described herein comprising the anti-PD-L1 antibody, anlotinib or the pharmaceutically acceptable salt thereof, and the chemotherapeutic drug comprises a pharmaceutical composition of the anti-PD-L1 antibody at an anti-PD-L1 antibody concentration of 10-60 mg/mL, and a pharmaceutical composition comprising anlotinib or the pharmaceutically acceptable salt thereof in a unit dose of 6 mg, 8 mg, 10 mg and/or 12 mg.

In some embodiments, the pharmaceutical combination described herein comprising the anti-PD-L1 antibody, anlotinib or the pharmaceutically acceptable salt thereof, and the chemotherapeutic drug comprises a pharmaceutical composition comprising 1200 mg of the anti-PD-L1 antibody provided in a multiple-dose form, and a pharmaceutical composition comprising anlotinib or the pharmaceutically acceptable salt thereof in a unit dose of 8 mg, 10 mg and/or 12 mg.

Further, the pharmaceutical combination described herein comprising the anti-PD-L1 antibody, anlotinib or the pharmaceutically acceptable salt thereof, and the chemotherapeutic drug is a formulation suitable for administration within a single treatment cycle (e.g., a treatment cycle of 21 days), and comprises a pharmaceutical composition comprising 600-2400 mg of the anti-PD-L1 antibody and a pharmaceutical composition comprising 84-168 mg of anlotinib. In some embodiments, the formulation described herein suitable for administration within a single treatment cycle (e.g., a treatment cycle of 21 days) further comprises a pharmaceutical composition comprising 90-1800 mg, preferably 150-1800 mg, of etoposide. In some embodiments, the formulation described herein suitable for administration within

a single treatment cycle (e.g., a treatment cycle of 21 days) further comprises a pharmaceutical composition comprising 50-800 mg of carboplatin.

Further, the pharmaceutical combination described herein comprising the anti-PD-L1 antibody, anlotinib or the pharmaceutically acceptable salt thereof, and the chemotherapeutic drug comprises the anti-PD-L1 antibody and anlotinib in a weight ratio of (0.35-29):1, preferably (3.5-29):1, more preferably (3.5-14.5):1, and most preferably (7-14.5):1. The anti-PD-L1 antibody and anlotinib are packaged either separately or together. Anlotinib can be packaged in multiple aliquots (e.g., 2 aliquots, 7 aliquots, 14 aliquots, 28 aliquots, or more); the anti-PD-L1 antibody can be packaged in a single aliquot or multiple aliquots (e.g., 2 aliquots, 4 aliquots, or more). In some embodiments, the pharmaceutical combination described herein comprises the anti-PD-L1 antibody, anlotinib or the pharmaceutically acceptable salt thereof, carboplatin, and etoposide, wherein the weight ratio of the anti-PD-L1 antibody, anlotinib or the pharmaceutically acceptable salt thereof, carboplatin, and etoposide is (300-1200):(42-84):(25-400):(45-900), for example, (300-1200):(42-84):(25-400):(75-900). Preferably, carboplatin and etoposide can be packaged in a single aliquot or in multiple aliquots (e.g., 3 aliquots, 6 aliquots, or more).

In another aspect, the present application further provides use of the pharmaceutical combination disclosed herein or the kit disclosed herein for preparing a medicament for use in treating small cell lung cancer in a patient. Alternatively, the present application further provides a method for treating small cell lung cancer, comprising administering to a patient in need an effective amount of the pharmaceutical combination disclosed herein or the kit disclosed herein. Alternatively, the present application further provides use of the pharmaceutical combination disclosed herein or the kit disclosed herein for treating small cell lung cancer in a patient. Alternatively, the present application further provides the pharmaceutical combination disclosed herein or the kit disclosed herein for use in treating small cell lung cancer in a patient.

In some embodiments, the method for treating small cell lung cancer disclosed herein comprises administering to the patient in need a therapeutically effective amount of an anti-PD-L1 antibody, anlotinib or a pharmaceutically acceptable salt thereof, and a chemotherapeutic drug. In some embodiments, the method comprises administering to the patient in need a first pharmaceutical combination in a first treatment phase; and optionally, administering to the patient in need a second pharmaceutical combination in a second treatment phase. In some embodiments, the first pharmaceutical combination comprises an anti-PD-L1 antibody, anlotinib or a pharmaceutically acceptable salt thereof, and a chemotherapeutic drug. In some embodiments, the second pharmaceutical combination comprises the anti-PD-L1 antibody and anlotinib or the pharmaceutically acceptable salt thereof.

In some embodiments, the first treatment phase comprises 1 to 10 treatment cycles, preferably 2 to 8 treatment cycles, and most preferably 4 treatment cycles.

In some embodiments, the chemotherapeutic drug is one or more selected from the group consisting of a platinum-based anti-tumor drug and a topoisomerase inhibitor. In some embodiments, the chemotherapeutic drug is selected from the group consisting of a platinum-based anti-tumor drug and etoposide. In some embodiments, the chemotherapeutic drug is selected from the group consisting of carboplatin and etoposide.

Furthermore, the anti-PD-L1 antibody, anlotinib or the pharmaceutically acceptable salt thereof, and the chemotherapeutic drug are each present in the form of a pharmaceutical composition, and can be administered simultaneously, sequentially, or intermittently. Still further, the anti-PD-L1 antibody is administered once every week, every 2 weeks, every 3 weeks, or every 4 weeks; preferably, the anti-PD-L1 antibody is administered at 600-2400 mg per dose. Still further, anlotinib is administered at a dose of 6 mg, 8 mg, 10 mg, or 12 mg once daily on an administration regimen of consecutively 2-week treatment and then 1-week interruption.

<u>Anlotinib</u>

As used herein, the chemical name of the free base of anlotinib is 1-[[[4-(4-fluoro-2-methyl-1*H*-indol-5-yl)oxy-6-methoxyquinolin-7-yl]oxy]methyl]cyclopropylatnine, which has the following structural formula:

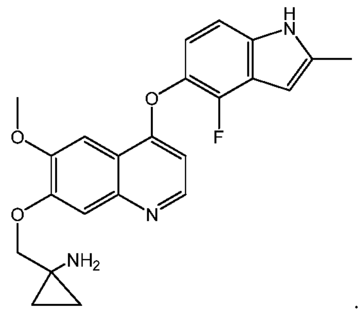

.

The pharmaceutically acceptable salt of anlotinib includes but is not limited to salts formed from anlotinib and an acid selected from the group consisting of the following: hydrochloric acid, hydrobromic acid, sulfuric acid, nitric acid, phosphoric acid, acetic acid, trifluoroacetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, *p*-toluenesulfonic acid, 3-phenylpropionic acid, trimethylacetic acid, *t*-butylacetic acid, dodecyl sulfuric acid, gluconic acid, glutamic acid, hydroxyl naphthoic acid, salicylic acid, and stearic acid; in some embodiments, the pharmaceutically acceptable salt is a hydrochloride or a maleate; in some embodiments, the pharmaceutically acceptable salt is a dihydrochloride.

Unless otherwise stated, the dose of anlotinib or the pharmaceutically acceptable salt thereof referred to in the present application is based on the molecular weight of the free base of anlotinib.

Anlotinib or the pharmaceutically acceptable salt thereof may be administered through various routes including gastrointestinal and parenteral administrations, or specifically, including, but not limited to, oral, intravenous, intra-arterial, transdermal, sublingual, intramuscular, rectal, transbuccal, intranasal, inhalational, vaginal, intraocular, subcutaneous, intra-adipose, intra-articular, intraperitoneal, and intrathecal administrations. In some specific embodiments, the drug is administered orally. The amount of anlotinib or the pharmaceutically acceptable salt thereof administered can be determined according to the severity of the disease, the response of the disease, any treatment-related toxicity, and the age and health of the patient. For example, anlotinib or the pharmaceutically acceptable salt thereof can be administered at a daily dose of 2 mg to 20 mg; in some embodiments, anlotinib or the pharmaceutically acceptable salt thereof can be administered at a daily dose of 2 mg, 3 mg, 4 mg, 5 mg, 6 mg, 7 mg, 8 mg, 9 mg, 10 mg, 11 mg, 12 mg, 13 mg, 14 mg, 15 mg, or 16 mg. Anlotinib or the pharmaceutically acceptable salt thereof can be administered once or multiple times daily. In some embodiments, anlotinib or the pharmaceutically acceptable salt thereof is administered once daily in the form of an oral solid formulation.

The administration regimen for anlotinib or the pharmaceutically acceptable salt thereof can be determined comprehensively depending on the activity and toxicity of the drug, the tolerance of the patient, and the like. Preferably, anlotinib or the pharmaceutically acceptable salt thereof is administered intermittently. The intermittent administration comprises a treatment period and an interruption period. Anlotinib or the pharmaceutically acceptable salt thereof can be administered once or multiple times daily in the treatment period. For example, the ratio of the treatment period to the interruption period in days is 2:(0.5-5), 2:(0.5-3), 2:(0.5-2), or 2:(0.5-1). In some embodiments, anlotinib or the pharmaceutically acceptable salt thereof is administered on a regimen of consecutively 2-week treatment and then 2-week interruption. In some embodiments, anlotinib or the pharmaceutically acceptable salt thereof is administered on a regimen of consecutively 2-week treatment and then 1-week interruption. In some embodiments, anlotinib or the pharmaceutically acceptable salt thereof is administered on a regimen of consecutively 5-day treatment and then 2-day interruption. For example, anlotinib or the pharmaceutically acceptable salt thereof can be administered orally at a dose of 6 mg, 8 mg, 10 mg, or 12 mg once daily on a regimen of consecutively 2-week treatment and then 1-week interruption.

Pharmaceutical Composition of Anlotinib or Pharmaceutically Acceptable Salt Thereof

In some embodiments of the present application, a unit dose of the pharmaceutical composition of anlotinib or the pharmaceutically acceptable salt thereof comprises 6 mg, 8 mg, 10 mg, or 12 mg of anlotinib.

In some embodiments of the present application, according to a treatment cycle of 2-week treatment and then 1-week interruption, the total dose of the pharmaceutical composition of anlotinib or the pharmaceutically acceptable salt thereof administered per cycle includes 84-168 mg. In some embodiments, the total dose of the pharmaceutical composition of anlotinib or the pharmaceutically acceptable salt thereof includes an amount selected from the group consisting of 84 mg, 112 mg, 140 mg and 168 mg or a range formed by any of the aforementioned values. In some embodiments, the total dose of the pharmaceutical composition of anlotinib or the pharmaceutically acceptable salt thereof includes 112-168 mg.

In some embodiments, the pharmaceutical composition includes but is not limited to formulations suitable for oral, parenteral, and local administrations; in some embodiments, the pharmaceutical composition is a formulation suitable for oral administration; in some embodiments, the pharmaceutical composition includes but is not limited to a tablet and a capsule.

Chemotherapeutic Drug

In some embodiments of the present application, the chemotherapeutic drug is one or more selected from the group consisting of a platinum-based anti-tumor drug and a topoisomerase inhibitor.

In the present application, the platinum-based anti-tumor drug includes but is not limited to one or more of cisplatin, carboplatin, nedaplatin, dicycloplatin, picoplatin, oxaliplatin, miriplatin, or lobaplatin. In some embodiments of

the present application, the platinum-based anti-tumor drug is selected from carboplatin.

In the present application, the topoisomerase inhibitor includes but is not limited to a topoisomerase I inhibitor, a topoisomerase II inhibitor, and a topoisomerase I/II dual inhibitor; in some embodiments, the topoisomerase inhibitor is selected from a topoisomerase II inhibitor. In some embodiments of the present application, the topoisomerase inhibitor includes but is not limited to one or more of camptothecin, topotecan, adriamycin, daunorubicin, epirubicin, idarubicin, mitoxantrone, irinotecan, topotecan, etoposide, or teniposide. In some embodiments of the present application, the topoisomerase inhibitor is selected from etoposide.

In some embodiments of the present application, the chemotherapeutic drug is selected from the group consisting of a platinum-based anti-tumor drug and etoposide. In some embodiments, the chemotherapeutic drug is selected from the group consisting of carboplatin and a topoisomerase inhibitor. In some embodiments of the present application, the chemotherapeutic drug is selected from the group consisting of carboplatin and/or etoposide. In some embodiments of the present application, the chemotherapeutic drug is selected from the group consisting of carboplatin and etoposide.

In some embodiments, carboplatin is administered at a dose corresponding to an AUC (area under curve) of 4-7 mg/mL/min, preferably at a dose corresponding to an AUC of 5-7 mg/mL/min, and more preferably at a dose corresponding to an AUC of 5 mg/mL/min.

In some embodiments, etoposide is administered at a dose of 60-120 mg/$m^2$, preferably at a dose of 60-100 mg/$m^2$, and more preferably at a dose of 100 mg/$m^2$. It will be appreciated, based on the general knowledge of those skilled in the art, that mg/$m^2$ refers to the dose of a drug used per square meter of body surface area of a subject.

Anti-PD-L1 Antibody

In some embodiments of the present application, the anti-PD-L1 antibody is the antibody disclosed in WO2016022630 or CN107001463A.

In some embodiments of the present application, the anti-PD-L1 antibody comprises the following amino acid sequences: a heavy chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to the amino acid sequence set forth in SEQ ID NO: 1 or SEQ ID NO: 4; a heavy chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to the amino acid sequence set forth in SEQ ID NO: 2 or SEQ ID NO: 5; a heavy chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to the amino acid sequence set forth in SEQ ID NO: 3 or SEQ ID NO: 6; a light chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to the amino acid sequence set forth in SEQ ID NO: 7 or SEQ ID NO: 10; a light chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to the amino acid sequence set forth in SEQ ID NO: 8 or SEQ ID NO: 11; and a light chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to the amino acid sequence set forth in SEQ ID NO: 9 or SEQ ID NO: 12.

In some embodiments of the present application, the anti-PD-L1 antibody comprises the following amino acid sequences: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12.

In some embodiments of the present application, the isolated anti-PD-L1 antibody described herein comprises: a heavy chain CDR1 region having the amino acid sequence set forth in SEQ ID NO: 1; a heavy chain CDR2 region having the amino acid sequence set forth in SEQ ID NO: 2; a heavy chain CDR3 region having the amino acid sequence set forth in SEQ ID NO: 3; a light chain CDR1 region having the amino acid sequence set forth in SEQ ID NO: 7; a light chain CDR2 region having the amino acid sequence set forth in SEQ ID NO: 8; and a light chain CDR3 region having the amino acid sequence set forth in SEQ ID NO: 9.

Each of the CDR regions described herein and the variants thereof described above are capable of specifically recognizing and binding to PD-L1, thereby effectively blocking the signaling between PD-L1 and PD-1.

In some embodiments of the present application, the anti-PD-L1 antibody comprises the following amino acid sequences: a heavy chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to the amino acid sequence set forth in SEQ ID NO: 13 or SEQ ID NO: 14; and a light chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to the amino

acid sequence set forth in SEQ ID NO: 15 or SEQ ID NO: 16.

In some embodiments of the present application, the anti-PD-L1 antibody comprises the following amino acid sequences: a heavy chain variable region set forth in SEQ ID NO: 13, and a light chain variable region set forth in SEQ ID NO: 15.

In some embodiments of the present application, the anti-PD-L1 antibody comprises the following amino acid sequences: a heavy chain variable region set forth in SEQ ID NO: 14, and a light chain variable region set forth in SEQ ID NO: 16.

In some embodiments of the present application, the anti-PD-L1 antibody comprises the following amino acid sequences: a heavy chain amino acid sequence set forth in SEQ ID NO: 17, and a light chain amino acid sequence set forth in SEQ ID NO: 18.

In some embodiments of the present application, the anti-PD-L1 antibody comprises the following amino acid sequences: a heavy chain amino acid sequence set forth in SEQ ID NO: 19, and a light chain amino acid sequence set forth in SEQ ID NO: 20.

In some embodiments of the present application, the anti-PD-L1 antibody comprises the following amino acid sequences: a heavy chain amino acid sequence set forth in SEQ ID NO: 21, and a light chain amino acid sequence set forth in SEQ ID NO: 18.

In one specific embodiment, the humanized anti-PD-L1 mAb disclosed herein comprises one or more conservatively substituted variants selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, and SEQ ID NO: 21. The humanized anti-PD-L1 mAb comprising the conservatively substituted variants retains the ability to specifically recognize and bind to PD-L1.

In some embodiments of the present application, the anti-PD-L1 antibody may be an IgG1 or IgG4 antibody.

In some embodiments of the present application, the anti-PD-L1 antibody is an IgG1 antibody. In some embodiments, the anti-PD-L1 antibody is a glycosylated IgG1 antibody.

In some embodiments of the present application, the anti-PD-L1 antibody comprises heavy chain complementarity determining regions (CDRs) selected from the group consisting of heavy chain CDRs derived from antibodies 13C5 and 5G11, and light chain CDRs selected from the group consisting of light chain CDRs derived from antibodies 13C5 and 5G11. In one embodiment, the anti-PD-L1 antibody described herein comprises: a heavy chain variable region of chimeric antibodies selected from the group consisting of ch5G11-hIgG1, ch5G11-hIgG4, ch13C5-hIgG1, and ch13C5-hIgG4; and a light chain variable region of chimeric antibodies selected from the group consisting of ch5G11-hIgG1, ch5G11-hIgG4, ch13C5-hIgG1, and ch13C5-hIgG4. In one embodiment, the anti-PD-L1 antibody described herein comprises: a heavy chain variable region of humanized antibodies selected from the group consisting of hu13C5-hIgG1, hu13C5-hIgG4, hu5G11-hIgG1, and hu5G11-hIgG4; and a light chain variable region of humanized antibodies selected from the group consisting of hu13C5-hIgG1, hu13C5-hIgG4, hu5G11-hIgG1, and hu5G11-hIgG4. Reference can be made to the description of Patent No. WO2016022630 or CN107001463A: 13C5, ch13C5-hIgG1, ch13C5-hIgG4, hu13C5-hIgG1, or hu13C5-hIgG4 comprises an HCDR1 sequence of SYGMS (SEQ ID NO: 4), an HCDR2 sequence of SISSGGSTYYPDSVKG (SEQ ID NO: 5), an HCDR3 sequence of GYDSGFAY (SEQ ID NO: 6), an LCDR1 sequence of ASQSVSTSSSSFMH (SEQ ID NO: 10), an LCDR2 sequence of YASNLES (SEQ ID NO: 11), and an LCDR3 sequence of QHSWEIPYT (SEQ ID NO: 12); and 5G11, ch5G11-hIgG1, ch5G11-hIgG4, hu5G11-hIgG1, or hu5G11-hIgG4 comprises an HCDR1 sequence of TYGVH (SEQ ID NO: 1), an HCDR2 sequence of VIWRGVTTDYNAAFMS (SEQ ID NO: 2), an HCDR3 sequence of LGFYAMDY (SEQ ID NO: 3), an LCDR1 sequence of KASQSVSNDVA (SEQ ID NO: 7), an LCDR2 sequence of YAANRYT (SEQ ID NO: 8), and an LCDR3 sequence of QQDYTSPYT (SEQ ID NO: 9).

In some embodiments of the present application, the number of species of the anti-PD-L1 antibody in the pharmaceutical combination may be selected from one or more. As used herein, the term "more" refers to more than one, for example, two, three, four, five, or more. For example, in some embodiments of the present application, the anti-PD-L1 antibody is selected from an antibody comprising a heavy chain variable region set forth in SEQ ID NO: 13 and a light chain variable region set forth in SEQ ID NO: 15, or selected from an antibody comprising a heavy chain variable region set forth in SEQ ID NO: 14 and a light chain variable region set forth in SEQ ID NO: 16, or selected from the group consisting of the combination thereof. As another example, the anti-PD-L1 antibody is selected from an antibody comprising a heavy chain amino acid sequence set forth in SEQ ID NO: 17 and a light chain amino acid sequence set forth in SEQ ID NO: 18, or selected from an antibody comprising a heavy chain amino acid sequence set forth in SEQ ID NO: 19 and a light chain amino acid sequence set forth in SEQ ID NO: 20, or selected from an antibody comprising a heavy chain amino acid sequence set forth in SEQ ID NO: 21 and a light chain amino acid sequence set forth in SEQ ID NO: 18, or selected from the group consisting of combinations of any of the foregoing.

In some embodiments, the anti-PD-L1 antibody comprises the following amino acid sequences: a heavy chain CDR1 region having at least 80% homology to the amino acid sequence set forth in SEQ ID NO: 1 or SEQ ID NO: 4; a heavy chain CDR2 region having at least 80% homology to the amino acid sequence set forth in SEQ ID NO: 2 or SEQ

ID NO: 5; a heavy chain CDR3 region having at least 80% homology to the amino acid sequence set forth in SEQ ID NO: 3 or SEQ ID NO: 6; a light chain CDR1 region having at least 80% homology to the amino acid sequence set forth in SEQ ID NO: 7 or SEQ ID NO: 10; a light chain CDR2 region having at least 80% homology to the amino acid sequence set forth in SEQ ID NO: 8 or SEQ ID NO: 11; and a light chain CDR3 region having at least 80% homology to the amino acid sequence set forth in SEQ ID NO: 9 or SEQ ID NO: 12.

Pharmaceutical Composition of Anti-PD-L1 Antibody

In some embodiments of the present application, the pharmaceutical composition of the anti-PD-L1 antibody comprises 600-2400 mg of the anti-PD-L1 antibody. In some embodiments, the pharmaceutical composition of the anti-PD-L1 antibody comprises the anti-PD-L1 antibody in an amount selected from the group consisting of 600 mg, 900 mg, 1200 mg, 1500 mg, 1800 mg, 2100 mg and 2400 mg or a range formed by any of the aforementioned values. In some embodiments, the pharmaceutical composition of the anti-PD-L1 antibody comprises 600-2100 mg or 900-1500 mg of the anti-PD-L1 antibody, wherein the pharmaceutical composition of the anti-PD-L1 antibody may be present in a multiple-dose or unit-dose form.

In some embodiments of the present application, the pharmaceutical composition of the anti-PD-L1 antibody comprises 300 mg, 600 mg, or 1200 mg of the anti-PD-L1 antibody. In some embodiments of the present application, provided is a pharmaceutical composition of the anti-PD-L1 antibody formulated into a unit dose comprising 300 mg, 600 mg, or 1200 mg of the anti-PD-L1 antibody.

In some embodiments, the pharmaceutical composition of the anti-PD-L1 antibody is a solution for injection. In some embodiments, the pharmaceutical composition of the anti-PD-L1 antibody is an aqueous solution for injection. In some embodiments of the present application, the pharmaceutical composition of the anti-PD-L1 antibody comprises one or more of a buffer, an isotonicity modifier, a stabilizer, and/or a surfactant. In particular, the pharmaceutical composition of the anti-PD-L1 antibody comprises the anti-PD-L1 antibody (e.g., mAb) at 1-150 mg/mL, the buffer at 3-50 mM, the isotonicity modifier/stabilizer at 2-150 mg/mL, and the surfactant at 0.01-0.8 mg/mL, and has a pH of 4.5-6.8.

In some embodiments of the present application, in the pharmaceutical composition of the anti-PD-L1 antibody, the anti-PD-L1 mAb is present at a concentration of 5-150 mg/mL (w/v); in some embodiments, the concentration is 10-60 mg/mL (w/v); in some embodiments, the concentration is 10-30 mg/mL (w/v). In some specific embodiments, the anti-PD-L1 mAb is present at a concentration of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, or 120 mg/mL (w/v); in some embodiments, the concentration is 10 mg/mL, 20 mg/mL, 30 mg/mL, 40 mg/mL, 50 mg/mL, or 60 mg/mL (w/v); in some embodiments, the concentration is 10 mg/mL, 20 mg/mL, or 30 mg/mL (w/v). In some embodiments, the anti-PD-L1 mAb is present at a concentration of 10 mg/mL (w/v). In other embodiments, the anti-PD-L1 mAb is present at a concentration of 30 mg/mL (w/v). In other embodiments, the anti-PD-L1 mAb is present at a concentration of 60 mg/mL (w/v).

In some embodiments of the present application, the buffer is a histidine salt buffer. The histidine salt buffer is present at a concentration of 5-30 mM; in some embodiments, the concentration is 10-25 mM; in some embodiments, the concentration is 10-20 mM; in some embodiments, the concentration is 10-15 mM. In some specific embodiments, the histidine salt buffer is present at a concentration of 5 mM, 10 mM, 15 mM, 20 mM, 25 mM, or 30 mM. In some embodiments, the histidine salt buffer is present at a concentration of 10 mM. In other embodiments, the histidine salt buffer is present at a concentration of 15 mM. In other embodiments, the histidine salt buffer is present at a concentration of 20 mM. The histidine salt buffer comprises histidine and hydrochloric acid.

In some embodiments of the present application, the isotonicity modifier/stabilizer is sucrose at 20-150 mg/mL (w/v); in some embodiments, the isotonicity modifier/stabilizer is sucrose at 40-100 mg/mL (w/v); in some embodiments, the isotonicity modifier/stabilizer is sucrose at 60-80 mg/mL (w/v). In some specific embodiments, the sucrose is present at a concentration of 40 mg/mL, 50 mg/mL, 60 mg/mL, 70 mg/mL, 80 mg/mL, 90 mg/mL, or 100 mg/mL. In some specific embodiments, the sucrose is present at a concentration of 60 mg/mL. In some specific embodiments, the sucrose is present at a concentration of 70 mg/mL. In some specific embodiments, the sucrose is present at a concentration of 80 mg/mL. In some specific embodiments, the sucrose is present at a concentration of 90 mg/mL.

In some embodiments of the present application, the surfactant is selected from the group consisting of polysorbate 80, polysorbate 20, and poloxamer 188; in some embodiments, the surfactant is selected from the group consisting of polysorbate 80 and polysorbate 20; in some embodiments, the surfactant is selected from polysorbate 80. In some embodiments, the surfactant is present at a concentration of 0.05-0.6 mg/mL (w/v); in some embodiments, the concentration is 0.1-0.4 mg/mL (w/v); in some embodiments, the concentration is 0.2-0.3 mg/mL (w/v).

In some embodiments of the present application, the surfactant is polysorbate 80 or polysorbate 20 at 0.01-0.8 mg/mL (w/v). In some specific embodiments, the surfactant is polysorbate 80 at 0.05-0.6 mg/mL; in some embodiments, the surfactant is polysorbate 80 at 0.1-0.4 mg/mL; in some embodiments, the surfactant is polysorbate 80 at 0.2-0.3 mg/mL; in some embodiments, the surfactant is polysorbate 80 at 0.2 mg/mL. In some embodiments, in the pharmaceutical composition, polysorbate 80 is present at a content of 0.1 mg/mL, 0.2 mg/mL, 0.3 mg/mL, 0.4 mg/mL, 0.5 mg/mL,

or 0.6 mg/mL; in some embodiments, in the pharmaceutical composition, polysorbate 80 is present at a content of 0.2 mg/mL, 0.3 mg/mL, 0.4 mg/mL, or 0.5 mg/mL; in some embodiments, in the pharmaceutical composition, polysorbate 80 is present at a content of 0.2 mg/mL, 0.3 mg/mL, or 0.4 mg/mL; in some embodiments, in the pharmaceutical composition, polysorbate 80 is present at a content of 0.2 mg/mL. In some embodiments, in the pharmaceutical composition, polysorbate 80 is present at a content of 0.1 mg/mL. In other embodiments, in the pharmaceutical composition, polysorbate 80 is present at a content of 0.2 mg/mL. In some embodiments, in the pharmaceutical composition, polysorbate 80 is present at a content of 0.3 mg/mL. In other embodiments, in the pharmaceutical composition, polysorbate 80 is present at a content of 0.4 mg/mL. In some embodiments, in the pharmaceutical composition, polysorbate 80 is present at a content of 0.5 mg/mL.

In some embodiments of the present application, the aqueous solution of the pharmaceutical composition has a pH selected from 4.0-6.8; in some embodiments, the pH is 4.5-6.5; in some embodiments, the pH is 5.5-6.0; in some embodiments, the pH is 5.5. In some embodiments, the aqueous solution of the pharmaceutical composition has a pH of 4.5, 4.8, 5.0, 5.2, 5.4, 5.5, 5.6, 5.8, or 6.0; in some embodiments, the pH is 5.0, 5.2, 5.4, 5.5, or 5.6; in some embodiments, the pH is 5.5. In some embodiments, the aqueous solution of the pharmaceutical composition has a pH of 5.0. In some embodiments, the aqueous solution of the pharmaceutical composition has a pH of 5.2. In some embodiments, the aqueous solution of the pharmaceutical composition has a pH of 5.4. In some embodiments, the aqueous solution of the pharmaceutical composition has a pH of 5.5. In some embodiments, the aqueous solution of the pharmaceutical composition has a pH of 5.6. In some embodiments, the aqueous solution of the pharmaceutical composition has a pH of 5.8. In some embodiments, the aqueous solution of the pharmaceutical composition has a pH of 6.0.

In some specific embodiments of the present application, the pharmaceutical composition comprises: (a) the anti-PD-L1 antibody at a concentration of 20 mg/mL (w/v), (b) sucrose at a concentration of 70 mg/mL (w/v), (c) polysorbate 80 at a concentration of 0.1 mg/mL (w/v), (d) histidine at a concentration of 20 mM, and (e) optionally hydrochloric acid of a suitable amount for adjusting the pH of the composition to 5.0. In one specific embodiment of the present application, the pharmaceutical composition comprises: (a) the anti-PD-L1 mAb at a concentration of 20 mg/mL (w/v), (b) sucrose at a concentration of 70 mg/mL (w/v), (c) polysorbate 80 at a concentration of 0.1 mg/mL (w/v), (d) histidine at a concentration of 20 mM, and (e) optionally hydrochloric acid of a suitable amount for adjusting the pH of the composition to 5.0.

In another specific embodiment of the present application, the pharmaceutical composition comprises: (a) the anti-PD-L1 antibody at a concentration of 10 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally hydrochloric acid of a suitable amount for adjusting the pH of the composition to 5.5.

In another specific embodiment of the present application, the pharmaceutical composition comprises: (a) the anti-PD-L1 antibody at a concentration of 50 mg/mL (w/v), (b) sucrose at a concentration of 80 mg/mL (w/v), (c) polysorbate 80 at a concentration of 0.3 mg/mL (w/v), (d) histidine at a concentration of 10 mM, and (e) optionally hydrochloric acid of a suitable amount for adjusting the pH of the composition to 5.5.

In another specific embodiment of the present application, the pharmaceutical composition comprises: (a) the anti-PD-L1 antibody at a concentration of 100 mg/mL (w/v), (b) sucrose at a concentration of 80 mg/mL (w/v), (c) polysorbate 80 at a concentration of 0.5 mg/mL (w/v), (d) histidine at a concentration of 10 mM, and (e) optionally hydrochloric acid of a suitable amount for adjusting the pH of the composition to 5.5.

In another specific embodiment of the present application, the pharmaceutical composition comprises: (a) the anti-PD-L1 antibody at a concentration of 30 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally hydrochloric acid of a suitable amount for adjusting the pH of the composition to 5.5.

In another specific embodiment of the present application, the pharmaceutical composition comprises: (a) the anti-PD-L1 antibody at a concentration of 60 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally hydrochloric acid of a suitable amount for adjusting the pH of the composition to 5.5.

In another specific embodiment of the present application, the pharmaceutical composition comprises: (a) the anti-PD-L1 antibody at a concentration of 10 mg/mL (w/v), (b) sucrose at a concentration of 70 mg/mL (w/v), (c) polysorbate 80 at a concentration of 0.4 mg/mL (w/v), (d) histidine at a concentration of 20 mM, and (e) optionally acetic acid of a suitable amount for adjusting the pH of the composition to 6.5.

In another specific embodiment of the present application, the pharmaceutical composition comprises: (a) the anti-PD-L1 mAb at a concentration of 10 mg/mL (w/v), (b) sucrose at a concentration of 80 mg/mL (w/v), (c) polysorbate 80 at a concentration of 0.2 mg/mL (w/v), (d) histidine at a concentration of 20 mM, and (e) optionally hydrochloric acid of a suitable amount for adjusting the pH of the composition to 5.5.

In another specific embodiment of the present application, the pharmaceutical composition is a water-soluble injection; in some embodiments, the water-soluble injection includes, but is not limited to, a water-soluble formulation without lyophilization or a water-soluble formulation reconstituted from a lyophilized powder. In other embodiments, the pharmaceutical composition is a lyophilized formulation. The lyophilized formulation refers to a formulation prepared by

subjecting an aqueous solution to a lyophilization process, in which a substance is first frozen, and then the amount of a solvent is reduced by sublimation (primary drying process) and then by desorption (secondary drying process) until the amount of the solvent is reduced to a value that no longer supports a biological activity or a chemical reaction. The lyophilized formulation of the present application can also be dried by other methods known in the art, such as spray drying and bubble drying.

Pharmaceutical Combination

In one aspect, the present application provides a pharmaceutical combination for use in treating small cell lung cancer, comprising an anti-PD-L1 antibody, anlotinib or a pharmaceutically acceptable salt thereof, and a chemotherapeutic drug.

In some embodiments of the present application, the pharmaceutical combination comprises the anti-PD-L1 antibody, anlotinib or the pharmaceutically acceptable salt thereof and the chemotherapeutic drug. In some embodiments of the present application, the pharmaceutical combination comprising the anti-PD-L1 antibody, anlotinib or the pharmaceutically acceptable salt thereof, and the chemotherapeutic drug is a fixed combination. In some embodiments, the fixed combination is in the form of a solid pharmaceutical composition or a liquid pharmaceutical composition. In some embodiments of the present application, the pharmaceutical combination comprising the anti-PD-L1 antibody, anlotinib or the pharmaceutically acceptable salt thereof and the chemotherapeutic drug is a non-fixed combination. In some embodiments, the anti-PD-L1 antibody, anlotinib or the pharmaceutically acceptable salt thereof, and the chemotherapeutic drug in the non-fixed combination are each present in the form of a pharmaceutical composition. In some embodiments, each active ingredient of the anti-PD-L1 antibody, anlotinib or the pharmaceutically acceptable salt thereof, and the chemotherapeutic drug in the non-fixed combination is present in the form of a pharmaceutical composition.

In some embodiments, provided is a pharmaceutical combination comprising the anti-PD-L1 antibody and anlotinib in a weight ratio of (0.35-29):1, preferably (3.5-29):1, more preferably (3.5-14.5):1, and most preferably (7-14.5):1. The anti-PD-L1 antibody and anlotinib are packaged either separately or together. Anlotinib can be packaged in multiple aliquots (e.g., 2 aliquots, 7 aliquots, 14 aliquots, 28 aliquots, or more).

In some embodiments of the present application, the pharmaceutical combination comprising the anti-PD-L1 antibody, anlotinib or the pharmaceutically acceptable salt thereof, and the chemotherapeutic drug comprises:

i) the anti-PD-L1 antibody, wherein in one embodiment, the anti-PD-L1 antibody comprises heavy chain complementarity determining regions (CDRs) selected from the group consisting of heavy chain CDRs derived from antibodies 13C5 and 5G11, and light chain CDRs selected from the group consisting of light chain CDRs derived from antibodies 13C5 and 5G11; in one embodiment, the anti-PD-L1 antibody comprises a heavy chain variable region of chimeric antibodies selected from the group consisting of ch5G11-hIgG1, ch5G11-hIgG4, ch13C5-hIgG1 and ch13C5-hIgG4, and a light chain variable region of chimeric antibodies selected from the group consisting of ch5G11-hIgG1, ch5G11-hIgG4, ch13C5-hIgG1 and ch13C5-hIgG4; in one embodiment, the anti-PD-L1 antibody comprises a heavy chain variable region of humanized antibodies selected from the group consisting of hu13C5-hIgG1, hu13C5-hIgG4, hu5G11-hIgG1 and hu5G11-hIgG4, and a light chain variable region of humanized antibodies selected from the group consisting of hu13C5-hIgG1, hu13C5-hIgG4, hu5G11-hIgG1 and hu5G11-hIgG4; in one embodiment, the anti-PD-L1 antibody is selected from the group consisting of CDRs including, e.g., the HCDR1 sequence (SYGMS), the HCDR2 sequence (SISSGGSTYYPDSVKG), the HCDR3 sequence (GYDSGFAY), the LCDR1 sequence (ASQSVSTSSSSFMH), the LCDR2 sequence (YASNLES) and the LCDR3 sequence (QHSWEIPYT) of antibodies 13C5, ch13C5-hIgG1, ch13C5-hIgG4, hu13C5-hIgG1 or hu13C5-hIgG4, and, the HCDR1 sequence (TYGVH), the HCDR2 sequence (VIWRGVTTDYNAAFMS), the HCDR3 sequence (LGFYAMDY), the LCDR1 sequence (KASQSVSNDVA), the LCDR2 sequence (YAANRYT) and the LCDR3 sequence (QQDYTSPYT) of antibodies 5G11, ch5G11-hIgG1, ch5G11-hIgG4, hu5G11-hIgG1 or hu5G11-hIgG4, as disclosed in Patent No. WO2016022630 or CN107001463A;

ii) anlotinib or the pharmaceutically acceptable salt thereof; and

iii) a platinum-based anti-tumor drug and/or etoposide.

In some embodiments of the present application, the pharmaceutical combination comprising the anti-PD-L1 antibody and the chemotherapeutic drug comprises a pharmaceutical composition comprising 600-2400 mg of the anti-PD-L1 antibody, a pharmaceutical composition comprising 50-700 mg of carboplatin, and a pharmaceutical composition comprising 20-200 mg of etoposide. The pharmaceutical composition of the anti-PD-L1 antibody, the pharmaceutical composition of carboplatin, and the pharmaceutical composition of etoposide are in a single dose or multiple doses.

In some embodiments of the present application, the pharmaceutical combination comprises the anti-PD-L1 antibody, anlotinib or the pharmaceutically acceptable salt thereof, and the chemotherapeutic drug; alternatively, the pharmaceutical combination comprises a pharmaceutical combination of the anti-PD-L1 antibody, anlotinib or the pharmaceutically acceptable salt thereof and the chemotherapeutic drug.

In some embodiments of the present application, the pharmaceutical combination comprising the anti-PD-L1 antibody, anlotinib or the pharmaceutically acceptable salt thereof, and the chemotherapeutic drug comprises a pharmaceutical composition of the anti-PD-L1 antibody, a pharmaceutical composition of anlotinib or the pharmaceutically acceptable salt thereof, and a pharmaceutical composition of the chemotherapeutic drug. In some embodiments of the present application, the pharmaceutical combination is packaged in a kit further comprising an instruction for the combined use of the anti-PD-L1 antibody, anlotinib or the pharmaceutically acceptable salt thereof, and the chemotherapeutic drug in treating small cell lung cancer.

In some embodiments of the present application, the pharmaceutical combination comprising the anti-PD-L1 antibody, anlotinib or the pharmaceutically acceptable salt thereof, and the chemotherapeutic drug comprises a pharmaceutical composition comprising 600-2400 mg of the anti-PD-L1 antibody, anlotinib or the pharmaceutically acceptable salt thereof in a single dose of 6 mg, 8 mg, 10 mg and/or 12 mg, a pharmaceutical composition comprising 50-800 mg (e.g., 50-500 mg) of carboplatin, and a pharmaceutical composition comprising 20-200 mg of etoposide. The pharmaceutical composition of the anti-PD-L1 antibody, the pharmaceutical composition of anlotinib or the pharmaceutically acceptable salt thereof, the pharmaceutical composition of carboplatin, and the pharmaceutical composition of etoposide are present in a single dose or multiple doses.

In some embodiments of the present application, the pharmaceutical combination comprising the anti-PD-L1 antibody, anlotinib or the pharmaceutically acceptable salt thereof, and the chemotherapeutic drug comprises a pharmaceutical composition comprising 1200 mg of the anti-PD-L1 antibody provided in a multiple-dose form, anlotinib in a single dose of 6 mg, 8 mg, 10 mg and/or 12 mg, a pharmaceutical composition comprising 200-400 mg of carboplatin, and a pharmaceutical composition comprising 50-150 mg of etoposide.

In some embodiments of the present application, the pharmaceutical combination comprising the anti-PD-L1 antibody, anlotinib or the pharmaceutically acceptable salt thereof, and the chemotherapeutic drug comprises a pharmaceutical composition of the anti-PD-L1 antibody comprising the anti-PD-L1 antibody at a concentration of 10-60 mg/mL, anlotinib in a single dose of 6 mg, 8 mg, 10 mg and/or 12 mg, a pharmaceutical composition comprising 50-500 mg of carboplatin, and a pharmaceutical composition comprising 20-200 mg of etoposide.

In some embodiments of the present application, the pharmaceutical combination comprising the anti-PD-L1 antibody, anlotinib or the pharmaceutically acceptable salt thereof, and the chemotherapeutic drug comprises a pharmaceutical composition of the anti-PD-L1 antibody comprising the anti-PD-L1 antibody at a concentration of 10 mg/mL, anlotinib in a single dose of 8 mg, 10 mg and/or 12 mg, a pharmaceutical composition comprising 200-400 mg of carboplatin, and a pharmaceutical composition comprising 50-150 mg of etoposide.

In some embodiments, the pharmaceutical combination comprises the anti-PD-L1 antibody and anlotinib in a weight ratio of (0.35-29):1, preferably (3.5-29): 1, more preferably (3.5-14.5):1, and most preferably (7-14.5):1. The anti-PD-L1 antibody and anlotinib are packaged either separately or together. Anlotinib can be packaged in multiple aliquots (e.g., 2 aliquots, 7 aliquots, 14 aliquots, 28 aliquots, or more).

In another aspect, the present application provides a pharmaceutical combination for use in treating small cell lung cancer, comprising a first pharmaceutical combination administered to a patient in need in the first treatment phase, and optionally a second pharmaceutical combination administered to a patient in need in the second treatment phase. In some embodiments of the present application, the first pharmaceutical combination may be one or more selected from the group consisting of the above pharmaceutical combinations.

In some embodiments, the first pharmaceutical combination comprises an anti-PD-L1 antibody, anlotinib or a pharmaceutically acceptable salt thereof, and a chemotherapeutic drug.

In some embodiments, the second pharmaceutical combination comprises the anti-PD-L1 antibody and anlotinib or the pharmaceutically acceptable salt thereof.

In some embodiments, the first treatment phase comprises 1 to 10 treatment cycles, preferably 2 to 8 treatment cycles, and most preferably 4 treatment cycles.

In some embodiments, the pharmaceutical combination further comprises a pharmaceutically acceptable carrier.

In some embodiments, the anti-PD-L1 antibody, anlotinib or the pharmaceutically acceptable salt thereof, and the chemotherapeutic drug in the pharmaceutical combination are each in the form of a pharmaceutical composition. In some embodiments, each active ingredient of the anti-PD-L1 antibody, anlotinib or the pharmaceutically acceptable salt thereof, and the chemotherapeutic drug in the pharmaceutical combination is present in the form of a pharmaceutical composition.

<u>Kit</u>

In another aspect, the present application provides a kit for use in treating small cell lung cancer, comprising a pharmaceutical composition of an anti-PD-L1 antibody and a pharmaceutical composition of anlotinib or a pharmaceutically acceptable salt thereof, and further comprises a chemotherapeutic drug and an instruction for the combined use thereof in treating small cell lung cancer. In some embodiments, provided is a kit for use in treating small cell lung cancer, comprising a pharmaceutical composition of the anti-PD-L1 antibody, a pharmaceutical composition of anlotinib or the pharmaceutically acceptable salt thereof, and/or a pharmaceutical composition of carboplatin and/or a pharmaceutical composition of etoposide, and an instruction for the combined use thereof in treating small cell lung cancer; alternatively, the kit comprises the pharmaceutical combination disclosed herein and an instruction for the combined use thereof in treating small cell lung cancer.

Use

In yet another aspect, the present application further provides use of the pharmaceutical combination disclosed herein or the kit disclosed herein for preparing a medicament for use in treating small cell lung cancer in a patient. The present application further provides a method for treating small cell lung cancer in a patient, comprising administering to a patient in need an effective amount of the pharmaceutical combination disclosed herein or the kit disclosed herein. The present application further provides use of the pharmaceutical combination disclosed herein or the kit disclosed herein for treating small cell lung cancer in a patient. The pharmaceutical combination or the kit is as described above.

In some embodiments of the present application, in the use or the method described above, the anti-PD-L1 antibody, anlotinib or the pharmaceutically acceptable salt thereof, and the chemotherapeutic drug are each present in the form of a pharmaceutical composition and can be administered simultaneously, sequentially or intermittently.

In some embodiments of the present application, in the use or the method described above, the anti-PD-L1 antibody, anlotinib or the pharmaceutically acceptable salt thereof, and the chemotherapeutic drug are each administered intermittently. In some embodiments, the anti-PD-L1 antibody, anlotinib or the pharmaceutically acceptable salt thereof, and the chemotherapeutic drug are each administered according to the same or different administration regimens. In some embodiments, the anti-PD-L1 antibody, anlotinib or the pharmaceutically acceptable salt thereof, and the chemotherapeutic drug are each administered according to different administration regimens.

In some embodiments of the present application, in the use or the method, the anti-PD-L1 antibody can be administered once every week (q1w), once every 2 weeks (q2w), once every 3 weeks (q3w), or once every 4 weeks (q4w). In one specific embodiment, the anti-PD-L1 antibody is administered once every 3 weeks. In some embodiments, the anti-PD-L1 antibody is administered at 600-2400 mg per dose.

In some embodiments of the present application, anlotinib or the pharmaceutically acceptable salt thereof can be administered at a dose of 6 mg, 8 mg, 10 mg or 12 mg once daily on a regimen of consecutively 2-week treatment and then 1-week interruption. In this case, the administration cycle comprises 3 weeks.

In some embodiments of the present application, the chemotherapeutic drug may be administered according to known administration regimens.

In some embodiments of the present application, the anti-PD-L1 antibody, anlotinib or the pharmaceutically acceptable salt thereof, and the chemotherapeutic drug each have the same or different administration cycles. In some specific embodiments, the anti-PD-L1 antibody, anlotinib or the pharmaceutically acceptable salt thereof, and the chemotherapeutic drug have the same administration cycle. For example, the administration cycle comprises 1 week, 2 weeks, 3 weeks, or 4 weeks.

In some embodiments of the present application, in the administration regimen of the pharmaceutical combination, or in the use or the method, the administration cycle comprises 21 days. The PD-L1 antibody is administered on day one of each administration cycle, and anlotinib or the pharmaceutically acceptable salt thereof and the chemotherapeutic drug are administered daily on days 1-14 of each administration cycle, wherein the chemotherapeutic drug can be administered according to known administration regimens. In one specific embodiment, the PD-L1 antibody is administered once on day one of each administration cycle, anlotinib or the pharmaceutically acceptable salt thereof is administered once daily on days 1-14 of each administration cycle, carboplatin is administered once on day 1 of each administration cycle, and/or etoposide is administered on days 1, 2, and 3 of each administration cycle.

In some embodiments of the present application, in the use or the method, the anti-PD-L1 antibody can be administered at a dose selected from the group consisting of 0.01 to 40 mg/kg, 0.1 to 30 mg/kg, 0.1 to 20 mg/kg, 0.1 to 15 mg/kg, 0.1 to 10 mg/kg, 1 to 15 mg/kg, 1 to 20 mg/kg, 1 to 3 mg/kg, 3 to 10 mg/kg, 3 to 15 mg/kg, 3 to 20 mg/kg, 3 to 30 mg/kg, 10 to 20 mg/kg, and 15 to 20 mg/kg, or administered to a patient at a dose of 60 mg to 2400 mg, 90 mg to about 1800 mg, 120 mg to 1500 mg, 300 mg to 900 mg, 600 mg to 900 mg, 300 mg to 1200 mg, 600 mg to 1200 mg, or 900 mg to 1200 mg.

In some embodiments for the use or the method, the administration cycle comprises 21 days; 1200 mg of the PD-L1 antibody is administered on day one of each administration cycle, and 6 mg, 8 mg, 10 mg and/or 12 mg of anlotinib is administered daily on days 1-14 of each administration cycle.

In some embodiments of the present application, in a three-week treatment cycle, the anti-PD-L1 antibody and anlotinib are administered to the subject in a weight ratio of (0.35-29): 1, preferably (3.5-29): 1, more preferably

(3.5-14.5):1, and most preferably (7-14.5):1, wherein the anti-PD-L1 antibody and anlotinib are administered in a single dose and multiple doses, respectively.

In some embodiments of the present application, in the administration regimen of the pharmaceutical combination, or in the use or the method, the anti-PD-L1 antibody, anlotinib or the pharmaceutically acceptable salt thereof, and the chemotherapeutic drug are administered for the treatment in a first treatment phase in repeated cycles; and optionally, the anti-PD-L1 antibody and anlotinib or the pharmaceutically acceptable salt thereof are administered for the treatment in a second treatment phase in repeated cycles. In some embodiments, for the first treatment phase and the second treatment phase, the administration cycle comprises 21 days. In some embodiments, in the first treatment phase, the PD-L1 antibody is administered on day one of each administration cycle, and anlotinib or the pharmaceutically acceptable salt thereof and the chemotherapeutic drug are administered daily on days 1-14 of each administration cycle, wherein the chemotherapeutic drug can be administered according to known administration regimens. In some embodiments, the chemotherapeutic drug is one or more selected from the group consisting of a platinum-based anti-tumor drug and/or a topoisomerase inhibitor. In some embodiments, the chemotherapeutic drug is selected from the group consisting of carboplatin and etoposide. In one specific embodiment, the PD-L1 antibody is administered once on day one of each administration cycle, anlotinib or the pharmaceutically acceptable salt thereof is administered once daily on days 1-14 of each administration cycle, carboplatin is administered once on day 1 of each administration cycle, and/or etoposide is administered on days 1, 2, and 3 of each administration cycle. In some embodiments, the first treatment phase comprises 1 to 10 treatment cycles, preferably 2 to 8 treatment cycles, and most preferably 4 treatment cycles.

In some embodiments, carboplatin is administered at a dose corresponding to an AUC of 4-7 mg/mL/min, preferably at a dose corresponding to an AUC of 5-7 mg/mL/min, and more preferably at a dose corresponding to an AUC of 5 mg/mL/min.

In some embodiments, etoposide is administered at a dose of 60-120 mg/m$^2$, preferably at a dose of 60-100 mg/m$^2$, and more preferably at a dose of 100 mg/m$^2$.

In some embodiments, in the second treatment phase, the PD-L1 antibody is administered on day one of each administration cycle, and anlotinib or the pharmaceutically acceptable salt thereof is administered daily on days 1-14 of each administration cycle.

Small Cell Lung Cancer

In some embodiments, the small cell lung cancer includes limited-stage and extensive-stage small cell lung cancer. It will be appreciated, based on the general knowledge of those skilled in the art, that the term "extensive-stage small cell lung cancer" includes, but is not limited to, untreated (i.e., previously untreated) extensive-stage small cell lung cancer.

In some embodiments, the small cell lung cancer is an advanced and/or refractory and/or recurrent and/or metastatic small cell lung cancer. In some embodiments, the small cell lung cancer includes sensitive relapsed and/or refractory relapsed small cell lung cancers. In some embodiments, the small cell lung cancer is a small cell lung cancer with brain metastasis.

In some embodiments of the present application, the patient with small cell lung cancer is a small cell lung cancer patient who has received surgery, chemotherapy, and/or radiotherapy. In some embodiments, the chemotherapy is selected from a chemotherapy comprising a platinum-based anti-tumor drug. In some embodiments, the platinum-based anti-tumor drug includes but is not limited to cisplatin and carboplatin.

In some embodiments, the small cell lung cancer is a small cell lung cancer that has not been previously treated with any systemic treatment. In some embodiments, the small cell lung cancer is a small cell lung cancer that has not been previously treated with any systemic treatment for extensive-stage small cell lung cancer. In some embodiments, the small cell lung cancer is an extensive-stage small cell lung cancer that has not been previously treated with any systemic treatment.

In some embodiments, the patient with small cell lung cancer is a patient who has previously received radiotherapy and/or chemotherapy for limited-stage small cell lung cancer.

The chemotherapeutic drug includes, but is not limited to, one or more of platinum-based anti-tumor drugs, podophyllums, alkylating agents, camptothecin analogs, taxanes, antimetabolites and anti-tumor antibiotics; examples that may be listed include, but are not limited to, one or more of platinum-based anti-tumor drugs (e.g., cisplatin, carboplatin, nedaplatin, miriplatin or oxaliplatin), etoposide, irinotecan, topotecan, paclitaxel, docetaxel, temozolomide, vinorelbine, gemcitabine, cyclophosphamide, adriamycin, vincristine, bendamustine, epirubicin, methotrexate, and amrubicin.

Administration

The content below is not intended to limit the administration of the pharmaceutical combination disclosed herein. The components in the pharmaceutical composition disclosed herein can be administered independently, or some or

all of the components are co-administered in various proper routes, including but not limited to oral administration or parenteral administration (by intravenous, intramuscular, local or subcutaneous routes). In some embodiments, the components in the pharmaceutical combination disclosed herein can be administered independently, or some or all of the components are co-administered by means of oral administration or injection, for example, intravenous injection or intraperitoneal injection.

The components in the pharmaceutical composition disclosed herein can be independent suitable dosage forms, or some or all of the components are co-formulated in a suitable dosage form, including, but not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, dispersant and dosage forms of sustained-release formulations for oral or non-oral administration.

The components in the pharmaceutical combination disclosed herein can be formulated independently, or some or all of the components are co-formulated with a pharmaceutically acceptable carrier and/or excipient.

The pharmaceutical combination disclosed herein may further comprise an additional therapeutic agent. In one embodiment, the additional therapeutic agent can be a known therapeutic agent for small cell cancer in the art.

## Administration Regimen

The administration regimen of the pharmaceutical combination disclosed herein or the kit disclosed herein comprises a first treatment phase and optionally a second treatment phase.

The administration regimen of the present application is also applicable to the use for preparing a medicament for use in treating small cell lung cancer, a method of treating small cell lung cancer in a patient, and the use for treating small cell lung cancer in a patient.

In some embodiments of the present application, the first treatment phase comprises 1-10 treatment cycles, preferably 2-8 treatment cycles, and most preferably 4 treatment cycles.

In some embodiments of the present application, the second treatment phase comprises 2-20 treatment cycles. In some embodiments, the second treatment phase continues until loss of clinical benefits, intolerable toxicity, PD upon efficacy assessment, or investigator-assessed ineligibility for further treatment.

In some embodiments of the present application, the treatment cycle comprises 14-42 days; in some embodiments, the treatment cycle comprises 14, 21, 28, 35, or 42 days; in some embodiments, the treatment cycle comprises 21 days. In some specific embodiments, the first treatment phase has the same treatment cycle as the second treatment phase. In some specific embodiments, the treatment cycles (e.g., a single treatment cycle) for both the first treatment phase and the second treatment phase comprise 21 days.

In some embodiments of the present application, the administration regimen for the first treatment phase comprises: 1) administering the anti-PD-L1 antibody once on one of days 1-7 of each treatment cycle; 2) administering anlotinib or the pharmaceutically acceptable salt thereof continuously on days 1-28 of each treatment cycle; optionally, 3) administering the platinum-based anti-tumor drug once on one of days 1-7 of each treatment cycle; and optionally, 4) administering the topoisomerase inhibitor continuously on days 1-7 of each treatment cycle.

In some embodiments, the treatment cycle comprises 21 days, and the administration regimen for the first treatment phase comprises: 1) administering the anti-PD-L1 antibody once on day 1 of each treatment cycle; 2) administering anlotinib or a pharmaceutically acceptable salt thereof continuously on days 1-14 of each treatment cycle; optionally, 3) administering the platinum-based anti-tumor drug once on day 1 of each treatment cycle; and optionally, 4) administering the topoisomerase inhibitor continuously on days 1-3 of each treatment cycle.

In some specific embodiments, the administration regimen for the first treatment phase comprises: 1) administering the anti-PD-L1 antibody once on one of days 1-7 of each treatment cycle; 2) administering anlotinib or the pharmaceutically acceptable salt thereof continuously on days 1-28 of each treatment cycle; 3) administering the platinum-based anti-tumor drug once on one of days 1-7 of each treatment cycle; and 4) administering the topoisomerase inhibitor continuously on days 1-7 of each treatment cycle.

In some embodiments of the present application, the treatment cycle comprises 21 days, and the administration regimen for the first treatment phase comprises: 1) administering the anti-PD-L1 antibody once on day 1 of each treatment cycle; 2) administering anlotinib or a pharmaceutically acceptable salt thereof continuously on days 1-14 of each treatment cycle; 3) administering the platinum-based anti-tumor drug once on day 1 of each treatment cycle; and 4) administering the topoisomerase inhibitor continuously on days 1-3 of each treatment cycle.

In some embodiments of the present application, the administration regimen for the first treatment phase comprises: 1) administering anti-PD-L1 antibody hu5G11-hIgG1 once on day 1 of each treatment cycle; 2) administering anlotinib dihydrochloride continuously on days 1-14 of each treatment cycle; 3) administering the platinum-based anti-tumor drug once on day 1 of each treatment cycle; and 4) administering the topoisomerase inhibitor continuously on days 1-3 of each treatment cycle.

In some embodiments of the present application, the administration regimen for the first treatment phase

comprises: 1) administering anti-PD-L1 antibody hu5G11-hIgG1 once on day 1 of each treatment cycle; 2) administering anlotinib dihydrochloride continuously on days 1-14 of each treatment cycle; 3) administering carboplatin once on day 1 of each treatment cycle; and 4) administering etoposide continuously on days 1-3 of each treatment cycle.

In some embodiments of the present application, the administration regimen for the second treatment phase comprises: 1) administering the anti-PD-L1 antibody once on one of days 1-7 of each treatment cycle; and optionally, 2) administering anlotinib or the pharmaceutically acceptable salt thereof continuously on days 1-28 of each treatment cycle.

In some embodiments of the present application, the administration regimen for the second treatment phase comprises: 1) administering the anti-PD-L1 antibody once on day 1 of each treatment cycle; and 2) administering anlotinib or a pharmaceutically acceptable salt thereof continuously on days 1-14 of each treatment cycle.

In some embodiments of the present application, the administration regimen for the second treatment phase comprises: 1) administering anti-PD-L1 antibody hu5G11-hIgG1 once on day 1 of each treatment cycle; and 2) administering anlotinib dihydrochloride continuously on days 1-14 of each treatment cycle.

In some embodiments of the present application, the anti-PD-L1 antibody is administered once on day 1, 2, 3, 4, 5, 6, or 7 of each treatment cycle, preferably once on day 1 of each treatment cycle.

In some embodiments of the present application, anlotinib or the pharmaceutically acceptable salt thereof is administered continuously on days 1-7, days 7-14, days 1-14, or days 7-21 of each treatment cycle, preferably on days 1-14 of each treatment cycle.

In some embodiments of the present application, the platinum-based anti-tumor drug is administered once on day 1, 2, 3, 4, 5, 6, or 7 of each treatment cycle, preferably once on day 1 of each treatment cycle.

In some embodiments of the present application, the topoisomerase inhibitor is administered continuously on days 1-3, days 1-4, days 1-5, days 1-6, days 1-7, days 2-4, days 2-5, days 2-6, days 2-7, days 3-5, days 3-6, days 3-7, days 4-6, days 4-7, or days 5-7 of each treatment cycle, preferably on days 1-3 of each treatment cycle.

## Technical Effects

The pharmaceutical combination of the present application can safely and effectively treat small cell lung cancer. In some embodiments, the pharmaceutical combination of the present application can safely and effectively treat extensive-stage small cell lung cancer. In some embodiments, the pharmaceutical combination of the present application can provide a treatment with higher tolerability in patients, and as compared with the administration of any one or two drugs of the combination, better anti-tumor effect and/or fewer adverse effects and/or complications. In some embodiments, the pharmaceutical combination of the present application exhibits a superior synergistic anti-tumor effect in the treatment of small cell lung cancer.

In patients with small cell lung cancer, particularly in patients with extensive-stage small cell lung cancer, the median PFS (progression-free survival), and/or the median OS (overall survival), and/or the OS were significantly prolonged after the treatment with the pharmaceutical combination disclosed herein is given. In some embodiments, the median PFS of the patients reaches 5-10 months after the treatment; in some specific embodiments, the median PFS of the patients is even greater than 10 months. In some embodiments, the median OS of the patients reaches 13-20 months after the treatment; in some specific embodiments, the median OS of the patients is even greater than 20 months.

After the patients with small cell lung cancer received the treatment with the pharmaceutical combination disclosed herein, the objective response rate (ORR) and the disease control rate (DCR) of the patients were significantly improved.

In one example of the present application, a 57-year-old patient was clinically diagnosed with extensive-stage small cell lung cancer in the lower portion of the right lung with metastasis to bilateral lung hila, mediastinum, bilateral supraclavicular lymph nodes, and the right middle lung lobe. After receiving 2 cycles (42 days, 3 weeks per cycle) of the study treatment with the anti-PD-L1 antibody hu5G11-hIgG1 injection, anlotinib hydrochloride capsules, carboplatin injection and etoposide injection described herein, the sum of target lesions (mass lesions in middle and lower lobes of the right lung and lymph nodes of left lung hilum) was 21 mm and was reduced by 65%, and the outcome was assessed as partial response (PR). After the patient continued the treatment according to the above protocol for another 2 cycles and then received maintenance treatment with the anti-PD-L1 antibody hu5G 11-hIgG 1 injection and anlotinib hydrochloride capsules, the target lesions were further reduced or disappeared. The outcome was assessed as partial response (PR). As of the data analysis, the progression-free survival (PFS) of the patient exceeded 27 months, and the patient was still on the treatment.

In another example of the present application, a 69-year-old patient was clinically diagnosed with small cell lung cancer in the lower portion of the left lung with metastasis to the left lower lung hilum and left lower lung lobe. After receiving 2 cycles (42 days, every 3 weeks per one treatment cycle) of the study treatment with the anti-PD-L1 antibody hu5G11-hIgG1 injection, anlotinib hydrochloride capsules, carboplatin injection and etoposide injection of the present application, the sum of target lesions (mass lesions in the lower portion of the left lung hilum and nodule lesions in lymph

nodes of mediastinum and the lower portion of the left lung) was 21 mm and was reduced by 79.4%, and the overall outcome was evaluated as partial response (PR). After the patient continued the treatment according to the above protocol for another 2 cycles and then received maintenance treatment with the anti-PD-L1 antibody hu5G11-hIgG1 injection and anlotinib hydrochloride capsules, the target lesions were further reduced or disappeared. The outcome was assessed as partial response (PR), and the progression-free survival (PFS) of the patient exceeded 20 months.

In yet another example of the present application, a 55-year-old patient was clinically diagnosed with small cell lung cancer in the left lung hilum with metastasis to lymph nodes of the mediastinum and bones. After receiving 2 cycles (42 days, every 3 weeks per one treatment cycle) of the study treatment with the anti-PD-L1 antibody hu5G 11-hIgG 1 injection, anlotinib hydrochloride capsules, carboplatin injection and etoposide injection of the present application, the sum of target lesions (mass lesion in the left lung hilum and lymph nodes of mediastinum) was 41 mm and was reduced by 39.7%, and the overall outcome was evaluated as partial response (PR). After the patient continued the treatment according to the above protocol for another 2 cycles and then received maintenance treatment with the anti-PD-L1 antibody hu5G11-hIgG1 injection and anlotinib hydrochloride capsules, the target lesions were further reduced or disappeared. The outcome was assessed as partial response (PR). As of the data analysis, the progression-free survival (PFS) of the patient exceeded 26 months, and the patient was still on the treatment.

Definitions and Explanations

Unless otherwise stated, the following terms used in the present application shall have the following meanings. A certain term, unless otherwise specifically defined, should not be construed as uncertain or unclear, but interpreted according to its common meaning in the field. When referring to a trade name, it is intended to refer to its corresponding commercial product or its active ingredient.

Herein, unless otherwise stated, the amount of anlotinib or a pharmaceutically acceptable salt thereof mentioned herein refers to the amount of the active ingredient anlotinib free base.

Unless otherwise specified, the term "dose" refers to a dose administered to a patient without considering the weight or the body surface area (BSA) of the patient. For example, a 60 kg human and a 100 kg human will receive the same dose of antibody (e.g., 240 mg of anti-PD-1 antibody).

As used herein, the term "pharmaceutical combination" refers to a combination of two or more active ingredients (administered as the respective active ingredients themselves, or as their respective derivatives like pharmaceutically acceptable salts or esters, prodrugs, or compositions) that are administered simultaneously or sequentially. The active substances can be administered to a subject simultaneously or sequentially in any order as individual formulations.

As used herein, the term "antibody" refers to a binding protein having at least one antigen-binding domain. The antibody and the fragment thereof of the present application can be an intact antibody or any fragment thereof. Thus, the antibody and the fragment thereof of the present application include a monoclonal antibody or a fragment thereof and an antibody variant or a fragment thereof, as well as an immunoconjugate. Examples of the antibody fragment include an Fab fragment, an Fab’ fragment, an F(ab)’ fragment, an Fv fragment, an isolated CDR region, a single-chain Fv molecule (scFv), an Fd fragment, and other antibody fragments known in the art. The antibody and the fragment thereof may also include a recombinant polypeptide, a fusion protein, and a bispecific antibody. The anti-PD-L1 antibody and the fragment thereof disclosed herein can be of IgG1, IgG2, IgG3, or IgG4 isotype. The term "isotype" refers to the class of antibodies encoded by the heavy chain constant region gene. In one embodiment, the anti-PD-L1 antibody and the fragment thereof disclosed herein are of the IgG1 or IgG4 isotype. The anti-PD-L1 antibody and the fragment thereof of the present application can be derived from any species including, but not limited to, mouse, rat, rabbit, primate, llama, and human. The anti-PD-L1 antibody and the fragment thereof can be a chimeric antibody, a humanized antibody, or an intact human antibody. In one embodiment, the anti-PD-L1 antibody is an antibody produced by a hybridoma cell line derived from a mouse. Thus, in one embodiment, the anti-PD-L1 antibody is a murine antibody. In another embodiment, the anti-PD-L1 antibody is a chimeric antibody. In another embodiment, the chimeric antibody is a mouse-human chimeric antibody. In another embodiment, the antibody is a humanized antibody. In another embodiment, the antibody is derived from a murine antibody and is humanized.

The term "humanized antibody" refers to an antibody comprising complementarity determining regions (CDRs) derived from a non-human antibody, and framework and constant regions derived from a human antibody. For example, an anti-PD-L1 antibody disclosed herein may comprise CDRs derived from one or more murine antibodies as well as human framework and constant regions. Thus, in one embodiment, the humanized antibody disclosed herein binds to the same epitope on PD-L1 as the murine antibody from which the CDRs of the humanized antibody are derived. Exemplary humanized antibodies are provided herein. Additional anti-PD-L1 antibodies or variants thereof comprising the heavy and light chain CDRs disclosed herein can be generated using any human framework sequences, and are also included in the present application. In one embodiment, framework sequences suitable for use in the present application include those similar in structure to the framework sequences disclosed herein. Additional modifications may be made in the framework regions to improve the properties of the antibodies provided herein. Such additional framework

modifications may include: chemical modifications, point mutations for reducing immunogenicity or removing T cell epitopes, or modifications reverting the mutations to residues in original germline sequences. In some embodiments, such modifications include those corresponding to the mutations exemplified herein, including reversions to germline sequences. For example, in one embodiment, one or more amino acids in the human VH and/or VL framework regions of the humanized antibodies disclosed herein are reverted to the corresponding amino acids in the parent murine antibodies. For example, for the VH and VL of humanized 5G11 and humanized 13C5 antibodies, several sites of framework amino acids of the template human antibodies described above are reverted to the corresponding amino acid sequences in the mouse 5G11 and 13C5 antibodies. In one embodiment, the amino acids at positions 53, 60, and/or 67 of the light chain variable region are reverted to the corresponding amino acids found at the positions in mouse 5G11 or 13C5 light chain variable region. In another embodiment, the amino acids at positions 24, 28, 30, 49, 73, 83, and/or 94 of the heavy chain variable region are reverted to the corresponding amino acids found at the positions in mouse 5G11 or 13C5 heavy chain variable region. In one embodiment, the humanized 5G11 antibody comprises: a light chain variable region, wherein the amino acid at position 60 is mutated from Ser (S) to Asp (D) and the amino acid at position 67 is mutated from Ser (S) to Tyr (Y); and a heavy chain variable region, wherein the amino acid at position 24 is mutated from Phe (F) to Val (V), the amino acid at position 49 is mutated from Ala (A) to Gly (G), the amino acid at position 73 is mutated from Thr (T) to Asn (N), and the amino acid at position 83 is mutated from Thr (T) to Asn (N). In one embodiment, the humanized 13C5 antibody comprises: a light chain variable region, wherein the amino acid at position 53 is mutated from Tyr (Y) to Lys (K); and a heavy chain variable region, wherein the amino acid at position 28 is mutated from Thr (T) to Ile (I), the amino acid at position 30 is mutated from Ser (S) to Arg (R), the amino acid at position 49 is mutated from Ser (S) to Ala (A), and the amino acid at position 94 is mutated from Tyr (Y) to Asp (D). Additional or alternative reverse mutations can be made in the framework regions of the humanized antibodies provided herein to improve the properties of the antibodies. The present application also encompasses humanized antibodies that bind to PD-L1 and comprise framework modifications corresponding to the exemplary modifications disclosed herein relative to any suitable framework sequence, as well as other framework modifications that otherwise improve antibody properties.

The present application provides an isolated antibody or a fragment thereof that binds to PD-L1, wherein the antibody can be produced by a hybridoma selected from the group consisting of the hybridomas designated herein as 13C5 and 5G11. Accordingly, the present application also encompasses hybridomas 13C5 and 5G11, and any hybridomas that produce the antibodies disclosed herein. The present application further provides an isolated polynucleotide encoding the antibody and the fragment thereof disclosed herein. The present application further encompasses an expression vector comprising the isolated polynucleotide, and a host cell comprising the expression vector.

The "isolated antibody" refers to an antibody that is substantially free of other antibodies having different antigenic specificities (e.g., an isolated antibody that specifically binds to PD-1 is substantially free of antibodies that specifically bind to antigens apart from PD-1). However, an isolated antibody that specifically binds to PD-1 may have cross-reactivity with other antigens (such as PD-1 molecules from different species). Furthermore, the isolated antibody may be substantially free of other cellular materials and/or chemicals.

The term "monoclonal antibody" ("mAb") refers to a non-native preparation of antibody molecules of a single molecule component (i.e., antibody molecules that have substantially identical base sequences and exhibit a single binding specificity and affinity for a particular epitope). mAb is an example of the isolated antibody. mAbs can be produced by hybridoma techniques, recombinant techniques, transgenic techniques, or other techniques known to those skilled in the art.

The antibody or the antigen-binding fragment thereof disclosed herein is specific for PD-L1. In one embodiment, the antibody or the fragment thereof is specific for PD-L1. In one embodiment, the antibody or the fragment thereof disclosed herein binds to human or primate PD-L1, but does not bind to PD-L1 from any other mammals. In another embodiment, the antibody or the fragment thereof does not bind to mouse PD-L1. The terms "human PD-L1", "hPD-L1", "huPD-L1", and the like are used interchangeably herein and refer to human PD-L1 and variants or isotypes of human PD-L1. The terms "specific", "specificity" and "specifically" refer to that the antibody and the fragment thereof bind to PD-L1 with greater affinity than any other targets.

The term "treat", "treating" or "treatment" usually refers to acquiring needed pharmacological effects and/or physiological effects. In terms of partially or fully stabilizing or curing a disease and/or an adverse effect of the disease, the effect can be therapeutic. As used herein, "treat", "treating" or "treatment" encompasses any treatment of a disease in a patient, including (a) inhibiting a symptom of the disease, i.e., blocking the progression of the disease; or (b) alleviating a symptom of the disease, i.e., causing the remission of the disease or the symptom.

The term "effective amount" refers to an amount of the compound disclosed herein for (i) treating a specific disease, condition, or disorder; (ii) alleviating, relieving, or eliminating one or more symptoms of a specific disease, condition, or disorder; or (iii) preventing or delaying the onset of one or more symptoms of a specific disease, condition, or disorder described herein. The amount of active substance (e.g., the antibody or compound disclosed herein) constituting the "therapeutically effective amount" may vary according to factors such as the disease state, age, sex, and weight of the individual, and the ability of a therapeutic agent or a combination of therapeutic agents to elicit a desired

response in the individual. The effective amount may also be determined routinely by those skilled in the art in accordance with their knowledge and the present disclosure.

The terms "administer", "administration" and "administering" are used interchangeably and refer to physically introducing the composition comprising a therapeutic agent to an entity using any of a variety of methods and delivery systems known to those skilled in the art. Routes of administration of immune checkpoint inhibitors (e.g., an anti-PD-1 antibody or an anti-PD-L1 antibody) include intravenous, intramuscular, subcutaneous, intraperitoneal, spinal, or other parenteral routes of administration, for example, by injection or infusion. As used herein, the phrase "parenteral administration" refers to modes of administration apart from enteral and local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and *in vivo* electroporation. In some embodiments, the immune checkpoint inhibitor (e.g., an anti-PD-1 antibody or an anti-PD-L1 antibody) is administered by a non-parenteral route, and in some embodiments, by oral administration. Other non-parenteral routes include local, epidermal or mucosal routes, for example, intranasal, vaginal, rectal, sublingual or local routes. Administration may also be performed, e.g., once, multiple times, and/or over one or more extended periods of time.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed by basic radicals and free acids and salts formed by acidic radicals and free bases, for example, hydrochloride, hydrobromide, nitrate, sulfate, phosphate, formate, acetate, trifluoroacetate, fumarate, oxalate, maleate, citrate, succinate, mesylate, benzenesulfonate and p-methylbenzenesulfonate, preferably, hydrochloride, hydrobromide, sulfate, formate, acetate, trifluoroacetate, fumarate, maleate, mesylate, p-methylbenzenesulfonate, sodium salt, potassium salt, ammonium salt, amino acid salt, *etc.* In the present application, when forming a pharmaceutically acceptable salt, the free acid and the basic radical are in a molar ratio of about 1:0.5 to 1:5, preferably 1:0.5, 1:1, 1:2, 1:3, 1:4, 1:5, 1:6, 1:7 or 1:8. In the present application, when forming a pharmaceutically acceptable salt, the free base and the acidic radical are in a molar weight ratio of about 1:0.5 to 1:5, preferably 1:0.5, 1:1, 1:2, 1:3, 1:4, 1:5, 1:6, 1:7 or 1:8.

As used herein, the terms "subject" and "patient" are used interchangeably. In some embodiments, the term "subject" or "patient" refers to a mammal. In some embodiments, the subject or patient is a mouse. In some embodiments, the subject or patient is a human.

The term "about" shall be interpreted as including a range of three standard deviations from the mean value or the standard tolerance range in a specific field. In some embodiments, "about" shall be interpreted as a variation not exceeding 0.5. The term "about" modifies all listed values thereafter. For example, "about 1, 2 and 3" represents "about 1", "about 2" and "about 3".

As used herein, "combined use" or "use in combination" means that two or more active substances may be administered to a patient simultaneously or sequentially in any order as individual formulations.

The term "single dose" refers to the smallest unit of packaging containing a certain quantity of pharmaceutical product; for example, in a box of seven capsules, each capsule is a single dose; or a vial of injection is a single dose.

The term "multiple doses" consists of multiple single doses.

The term "fixed combination" refers to the administration of the active components (for example, the anti-PD-L1 antibody and anlotinib) to a patient simultaneously at a fixed total dose or in a fixed dose ratio, or in the form of a single substance, pharmaceutical composition, or formulation.

The term "non-fixed combination" refers to simultaneous, parallel or sequential and temporally unlimited administration of two or more aforementioned active components as independent substances (for example, a pharmaceutical composition and a formulation) to a patient, wherein the active ingredients administered to the patient reach therapeutically effective amounts. An example, which can be listed, of the non-fixed combination is a cocktail therapy, for example, 3 or more active components are administered. In a non-fixed combination, each active ingredient can be packaged, sold or administered as a fully independent pharmaceutical composition. The term "non-fixed combination" also includes combined use of "fixed combinations", or a "fixed combination" and an independent substance of any one or more active components.

The term "pharmaceutical composition" refers to a mixture consisting of one or more of the active ingredients or pharmaceutical combinations thereof disclosed herein and a pharmaceutically acceptable excipient. The pharmaceutical composition is intended to facilitate the administration of the compound or the pharmaceutical combination thereof disclosed herein to a patient.

As used herein, unless otherwise stated, the terms "comprise", "comprises" and "comprising" or equivalents thereof are open-ended statements and mean that elements, components, and steps that are not specified may be included in addition to those listed.

Unless otherwise specified clearly herein, singular terms encompass plural referents, and vice versa. Similarly, unless otherwise specified clearly herein, the word "or" is intended to include "and", and vice versa.

All patents, patent applications, and other publications are explicitly incorporated herein by reference for the purpose of description and disclosure. These publications are provided solely because they were disclosed prior to the filing date of the present application. All statements as to the dates of these documents or descriptions as to the contents of these documents are based on the information available to the applicant and do not constitute any admission as to the correctness of the dates or the content of these documents. Moreover, in any country or region, any reference to these publications herein shall not be construed as an admission that the publications form part of the common knowledge in the art.

## DETAILED DESCRIPTION

For clarity, the present application is further described with the following examples, which are, however, not intended to limit the scope of the present application. All reagents used in the present application are commercially available and can be used without further purification. In the examples, the anti-PD-L1 antibody was prepared as described in WO2016022630, and after affinity chromatography, an eluate containing the antibody was obtained by conventional antibody purification methods.

### Example 1 Clinical trial

Eligible subjects were randomized to treatment group 1, treatment group 2, or control group. The treatment was divided into an induction treatment phase and a maintenance treatment phase. The efficacy was assessed every 2 cycles. For patients with disease under control (CR + PR + SD) and tolerable adverse reactions, the administration could be continued. The treatment was discontinued until loss of clinical benefits, intolerable toxicity, PD upon efficacy assessment, or investigator-assessed ineligibility for further treatment.

1.1 Primary inclusion criteria

1) Patients with pathologically confirmed extensive-stage small cell lung cancer (staged according to the Veterans Administration Lung Study Group (VALG));
2) Patients not previously treated with systemic treatment intended for extensive-stage small cell lung cancer;
3) Patients who have previously received radiotherapy and chemotherapy for limited-stage SCLC, must have received treatment with radical therapy, and have had a treatment-free intermittent period of at least 6 months between the end of chemotherapy, radiotherapy or radiotherapy and chemotherapy and the diagnosis of extensive-stage SCLC;
4) The presence of at least one measurable lesion according to RECIST 1.1;
5) Aged 18-75 years with an ECOG score of 0-1 and an expected survival of more than 3 months.

1.2 Study drugs

Anti-PD-L1 antibody hu5G11-hIgG1 injection or placebo injection: 1200 or 0 mg of anti-PD-L1 antibody injection was diluted to 250 mL with normal saline; the dilution was administered in 60 $\pm$ 10 min by infusion; the infusion system was rinsed with normal saline according to the routine procedures of the hospital after the completion of infusion; the treatment was given once on day 1 of every 21 days (one treatment cycle). Strength: 100 mg/10 mL, 300 mg/10 mL.

Anlotinib hydrochloride capsule (active ingredient: anlotinib dihydrochloride) or placebo capsule: one anlotinib hydrochloride capsule (12 mg) or one placebo capsule (0 mg) was administered orally at fasting once daily; the treatment was given for 2 weeks and interrupted for 1 week, i.e., the capsules were administered on days 1-14 of each treatment cycle (every 21 days). Strength: 12 mg, 10 mg, and 8 mg.

Carboplatin injection: the product was dissolved in 5% glucose injection at a concentration of 10 mg/mL; the solution was then added to 250-500 mL of 5% glucose injection for intravenous infusion on day 1 of each treatment cycle (every 21 days).

$$\text{Dose (mg)} = 5\ \text{(AUC) (mg/mL/min)} \times [\text{creatinine clearance rate within 7 days pre-dose (mL/min)} + 25].$$

The creatinine clearance rate (CCr) (unit: mL/min) was calculated according to the serum creatinine (SCr)

value, and the calculation formula was CCr = (140 - age) × weight (kg)/[72 × SCr (mg/dL)], or CCr = [(140 - age) × weight (kg)]/[0.818 × SCr (μmol/L)]. The unit of creatinine should be noted during the calculation of the creatinine clearance rate, and the calculation result should be multiplied by 0.85 for a female patient.

Etoposide injection: after the dose was calculated by the calculation formula, the product was diluted in sodium chloride injection at a concentration not exceeding 0.25 mg per milliliter. The treatment was given on days 1, 2, and 3 of each treatment cycle (every 21 days). Dose: 100 mg/$m^2$.

1.3 Administration regimen

The treatment is divided into an induction treatment phase (i.e., the first treatment phase) and a maintenance phase (i.e., the second treatment phase).

The first treatment phase was 4 treatment cycles:

Treatment group 1: The treatments were given on 21-day treatment cycles.

The anti-PD-L1 antibody injection was intravenously infused at 1200 mg/dose once every 21 days;

The anlotinib hydrochloride capsules were administered orally at 12 mg/dose for 2 weeks and interrupted for 1 week;

Carboplatin for injection was intravenously infused as per an AUC of 5 mg/mL/min (at a maximum dose of 800 mg) on the day 1 of each treatment cycle;

Etoposide injection was intravenously infused on days 1, 2, and 3 at 100 mg/$m^2$.

Treatment group 2: The treatments were given on 21-day treatment cycles.

The placebo injection for anti-PD-L1 antibody was intravenously infused at 0 mg/dose once every 21 days;

The anlotinib hydrochloride capsules were administered orally at 12 mg/dose for 2 weeks and interrupted for 1 week;

Carboplatin for injection was intravenously infused as per an AUC of 5 mg/mL/min (at a maximum dose of 800 mg) on the day 1 of each treatment cycle;

Etoposide injection was intravenously infused on days 1, 2, and 3 at 100 mg/$m^2$.

Control group: The treatments were given on 21-day treatment cycles.

The placebo injection for anti-PD-L1 antibody was intravenously infused at 0 mg/dose once every 21 days;

Placebo capsules for anlotinib hydrochloride were administered orally at 0 mg/dose for 2 weeks and interrupted for 1 week;

Carboplatin for injection was intravenously infused as per an AUC of 5 mg/mL/min (at a maximum dose of 800 mg) on the day 1 of each treatment cycle;

Etoposide injection was intravenously infused on days 1, 2, and 3 at 100 mg/$m^2$.

Second treatment phase (until loss of clinical benefits, intolerable toxicity, PD upon efficacy assessment, or investigator-assessed ineligibility for further treatment):

Treatment group 1: The treatments were given on 21-day treatment cycles.

The anti-PD-L1 antibody injection was intravenously infused at 1200 mg/dose once every 21 days;

The anlotinib hydrochloride capsules were administered orally at 12 mg/dose for 2 weeks and interrupted for 1 week;

Treatment group 2: The treatments were given on 21-day treatment cycles.

The placebo injection for anti-PD-L1 antibody was intravenously infused at 0 mg/dose once every 21 days;

The anlotinib hydrochloride capsules were administered orally at 12 mg/dose for 2 weeks and interrupted for 1 week;

Control group: The treatments were given on 21-day treatment cycles.

The placebo injection for anti-PD-L1 antibody was intravenously infused at 0 mg/dose once every 21 days;

Placebo capsules for anlotinib hydrochloride were administered orally at 0 mg/dose for 2 weeks and interrupted for 1 week.

1.3 Assessment

1) Safety assessment: the adverse events of the test treatment were assessed according to the NCI-CTCAE 5.0 criteria.
2) Efficacy assessment: the disease status was determined according to the RECIST 1.1/iRECIST criteria, and mainly according to RECIST 1.1 criteria.

1.4 Endpoints

Primary endpoints: progression-free survival (PFS) and overall survival (OS);

Secondary endpoints: objective response rate (ORR; complete response (CR) + partial response (PR)), disease control rate (DCR; CR + PR + stable disease (SD)), progression-free survival (PFS), overall survival (OS), duration of response (DOR), and the like. Incidence and severity of adverse events (AEs) and serious adverse events (SAEs), and abnormal laboratory test indicators.

1.5 Results

As of the data analysis, over 500 subjects were enrolled in the study. According to the current overall study data, subjects were expected to acquire significant clinical benefits by receiving the treatment of the treatments in treatment groups 1 and 2 as compared to the conventional first-line standard chemotherapy regimen (i.e., the control group). In addition, the improvement in the current overall efficacy data suggested that the median PFS of the subjects in treatment group 1 was significantly improved, and the median OS was expected to exceed 14 months. It is demonstrated that the combination of the anti-PD-L1 antibody, anlotinib or the pharmaceutically acceptable salt thereof, and the chemotherapeutic drug may be a more effective first-line treatment regimen for small cell lung cancer, especially for extensive-stage small cell lung cancer.

Those skilled in the art will appreciate that the scope of the present application is not limited to the embodiments and examples described above. Instead, various modifications, substitutions, or recombinations can be made without departing from the spirit of the present application, all of which fall within the protection scope of the present application.

## Claims

1. Use of a pharmaceutical combination comprising an anti-PD-L1 antibody, anlotinib or a pharmaceutically acceptable salt thereof, and a chemotherapeutic drug for preparing a medicament for treating small cell lung cancer.

2. The use according to claim 1, wherein the pharmaceutical combination comprises a first pharmaceutical combination and optionally a second pharmaceutical combination; the first pharmaceutical combination comprises the anti-PD-L1 antibody, anlotinib or the pharmaceutically acceptable salt thereof, and the chemotherapeutic drug.

3. The use according to claim 2, wherein the second pharmaceutical combination comprises the anti-PD-L1 antibody and anlotinib or the pharmaceutically acceptable salt thereof.

4. The use according to claim 2 or 3, wherein the first pharmaceutical combination is administered to a patient in need in a first treatment phase, and optionally the second pharmaceutical combination is administered to the patient in need in a second treatment phase.

5. The use according to claim 4, wherein the first treatment phase comprises 1 to 10 treatment cycles, preferably 2 to 8 treatment cycles, and most preferably 4 treatment cycles.

6. The use according to any one of claims 1-5, wherein the chemotherapeutic drug is one or more selected from the group consisting of a platinum-based anti-tumor drug and a topoisomerase inhibitor.

7. The use according to claim 6, wherein the platinum-based anti-tumor drug is one or more selected from the group consisting of cisplatin, carboplatin, nedaplatin, dicycloplatin, picoplatin, oxaliplatin, miriplatin, or lobaplatin.

8. The use according to claim 6 or 7, wherein the topoisomerase inhibitor is one or more selected from the group consisting of camptothecin, topotecan, adriamycin, daunorubicin, epirubicin, idarubicin, mitoxantrone, irinotecan, etoposide, or teniposide.

9. The use according to any one of claims 1-8, wherein the chemotherapeutic drug is selected from the group consisting of a platinum-based anti-tumor drug and etoposide, and preferably selected from the group consisting of carboplatin and etoposide.

10. The use according to any one of claims 1-9, wherein the anti-PD-L1 antibody is prepared into a pharmaceutical composition, and the anti-PD-L1 antibody in the pharmaceutical composition is present at a concentration of 10-60 mg/mL, or 10 mg/mL, 20 mg/mL, 30 mg/mL, 40 mg/mL, 50 mg/mL or 60 mg/mL.

11. The use according to any one of claims 1-10, wherein the pharmaceutical combination is a formulation suitable for administration within a single treatment cycle (e.g., a treatment cycle of 21 days), and comprises: a pharmaceutical composition comprising 600-2400 mg of the anti-PD-L1 antibody and a pharmaceutical composition comprising 84-168 mg of anlotinib or the pharmaceutically acceptable salt thereof.

12. The use according to any one of claims 9-11, wherein the formulation suitable for administration within a single treatment cycle further comprises: a pharmaceutical composition comprising 90-1800 mg of etoposide, and/or a pharmaceutical composition comprising 50-800 mg of carboplatin.

13. The use according to any one of claims 1-12, wherein the daily dose of the anti-PD-L1 antibody or the pharmaceutical composition thereof is 600 mg, 800 mg, 1000 mg, 1200 mg, 1400 mg, 1600 mg, 1800 mg, 2000 mg, 2200 mg, or 2400 mg, and the daily dose of anlotinib or the pharmaceutically acceptable salt thereof is 6-12 mg, or 6 mg, 8 mg, 10 mg or 12 mg.

14. The use according to any one of claims 9-13, wherein etoposide is administered at a dose of 60-100 mg/$m^2$, and preferably administered at a dose of 100 mg/$m^2$.

15. The use according to any one of claims 9-14, wherein carboplatin is administered at a dose corresponding to an AUC of 5-7 mg/mL/min, and preferably administered at a dose corresponding to an AUC of 5 mg/mL/min.

16. The use according to any one of claims 1-15, wherein the anti-PD-L1 antibody, anlotinib or the pharmaceutically acceptable salt thereof, and the chemotherapeutic drug are each present in the form of a pharmaceutical composition and can be administered simultaneously, non-simultaneously, or sequentially.

17. The use according to any one of claims 1-16, wherein the treatment cycle comprises 1 week, 2 weeks, 3 weeks, or 4 weeks.

18. The use according to any one of claims 1-17, wherein the treatment cycle comprises 3 weeks; the anti-PD-L1 antibody is administered on day 1 of each cycle, and anlotinib or the pharmaceutically acceptable salt thereof is administered on days 1-14 of each cycle.

19. The use according to any one of claims 9-18, wherein the treatment cycle comprises 3 weeks; etoposide is administered on days 1-3 of each cycle, and carboplatin is administered on day 1 of each cycle.

**20.** The use according to any one of claims 1-19, wherein the small cell lung cancer includes limited-stage and extensive-stage small cell lung cancers; or

the small cell lung cancer is a small cell lung cancer that has not previously been treated with any systemic treatment, and preferably the small cell lung cancer is an extensive-stage small cell lung cancer that has not previously been treated with any systemic treatment; or
the small cell lung cancer is an advanced and/or refractory and/or recurrent and/or metastatic small cell lung cancer.

**21.** A method for treating small cell lung cancer, comprising: administering to a patient in need a therapeutically effective amount of an anti-PD-L1 antibody, anlotinib or a pharmaceutically acceptable salt thereof, and a chemotherapeutic drug.

**22.** The method according to claim 21, comprising: administering to a patient in need a first pharmaceutical combination during a first treatment phase, and optionally administering to the patient in need a second pharmaceutical combination during a second treatment phase, wherein the first pharmaceutical combination comprises the anti-PD-L1 antibody, anlotinib or the pharmaceutically acceptable salt thereof, and the chemotherapeutic drug.

**23.** The method according to claim 22, wherein the second pharmaceutical combination comprises the anti-PD-L1 antibody and anlotinib or the pharmaceutically acceptable salt thereof.

**24.** The method according to claim 22 or 23, wherein the first treatment phase comprises 1 to 10 treatment cycles, preferably 2 to 8 treatment cycles, and most preferably 4 treatment cycles.

**25.** The method according to any one of claims 21-24, wherein the chemotherapeutic drug is one or more selected from the group consisting of a platinum-based anti-tumor drug and a topoisomerase inhibitor.

**26.** The method according to any one of claims 21-25, wherein the chemotherapeutic drug is selected from the group consisting of a platinum-based anti-tumor drug and etoposide, and preferably selected from the group consisting of carboplatin and etoposide.

**27.** The method according to any one of claims 21-26, wherein the anti-PD-L1 antibody, anlotinib or the pharmaceutically acceptable salt thereof, and the chemotherapeutic drug are each present in the form of a pharmaceutical composition and can be administered simultaneously, non-simultaneously, or sequentially.

**28.** The method according to any one of claims 21-27, wherein the daily dose of the anti-PD-L1 antibody or the pharmaceutical composition thereof is 600-2400 mg, or 600 mg, 800 mg, 1000 mg, 1200 mg, 1400 mg, 1600 mg, 1800 mg, 2000 mg, 2200 mg or 2400 mg.

**29.** The method according to any one of claims 21-28, wherein the daily dose of anlotinib or the pharmaceutically acceptable salt thereof or the pharmaceutical composition thereof is 6-12 mg, or 6 mg, 8 mg, 10 mg or 12 mg.

**30.** The method according to any one of claims 26-29, wherein etoposide is administered at a dose of 60-100 mg/$m^2$, and preferably administered at a dose of 100 mg/$m^2$.

**31.** The method according to any one of claims 26-30, wherein carboplatin is administered at a dose corresponding to an AUC of 5-7 mg/mL/min, and preferably administered at a dose corresponding to an AUC of 5 mg/mL/min.

**32.** The method according to any one of claims 21-31, wherein the treatment cycle comprises 1 week, 2 weeks, 3 weeks, or 4 weeks.

**33.** The method according to any one of claims 21-32, wherein the treatment cycle comprises 3 weeks; the anti-PD-L1 antibody is administered on day 1 of each cycle, and anlotinib or the pharmaceutically acceptable salt thereof is administered on days 1-14 of each cycle.

**34.** The method according to any one of claims 26-33, wherein the treatment cycle comprises 3 weeks; etoposide is administered on days 1-3 of each cycle, and carboplatin is administered on day 1 of each cycle.

**35.** The method according to any one of claims 21-34, wherein the small cell lung cancer includes limited-stage and extensive-stage small cell lung cancers; or

the small cell lung cancer is a small cell lung cancer that has not previously been treated with any systemic treatment, and preferably the small cell lung cancer is an extensive-stage small cell lung cancer that has not previously been treated with any systemic treatment; or
the small cell lung cancer is an advanced and/or refractory and/or recurrent and/or metastatic small cell lung cancer.

**36.** A pharmaceutical combination for treating small cell lung cancer, comprising an anti-PD-L1 antibody, anlotinib or a pharmaceutically acceptable salt thereof, and a chemotherapeutic drug.

**37.** The pharmaceutical combination according to claim 36, comprising a first pharmaceutical combination and optionally a second pharmaceutical combination, wherein the first pharmaceutical combination comprises the anti-PD-L1 antibody, anlotinib or the pharmaceutically acceptable salt thereof, and the chemotherapeutic drug.

**38.** The pharmaceutical combination according to claim 37, wherein the second pharmaceutical combination comprises the anti-PD-L1 antibody and anlotinib or the pharmaceutically acceptable salt thereof.

**39.** The pharmaceutical combination according to any one of claims 36-38, wherein the chemotherapeutic drug is one or more selected from the group consisting of a platinum-based anti-tumor drug and/or a topoisomerase inhibitor, preferably the chemotherapeutic drug is selected from the group consisting of a platinum-based anti-tumor drug and etoposide, and more preferably the chemotherapeutic drug is selected from the group consisting of carboplatin and etoposide.

**40.** The pharmaceutical combination according to any one of claims 36-39, wherein the anti-PD-L1 antibody, anlotinib or the pharmaceutically acceptable salt thereof, and the chemotherapeutic drug are each present in the form of a pharmaceutical composition.

**41.** The pharmaceutical combination according to any one of claims 36-40, comprising: a pharmaceutical composition comprising 600-2400 mg of the anti-PD-L1 antibody provided in a multiple-dose form, and a pharmaceutical composition comprising anlotinib or the pharmaceutically acceptable salt thereof in a unit dose of 6 mg, 8 mg, 10 mg and/or 12 mg.

**42.** The pharmaceutical combination according to any one of claims 39-41, further comprising: the pharmaceutical composition comprising 90-1800 mg of etoposide, and/or the pharmaceutical composition comprising 50-800 mg of carboplatin.

**43.** The pharmaceutical combination according to any one of claims 36-42, wherein the pharmaceutical combination is packaged in a kit further comprising an instruction for the treatment of small cell lung cancer.

**44.** The use according to any one of claims 1-20, or the method according to any one of claims 21-35, or the pharmaceutical combination according to any one of claims 36-43, wherein the anti-PD-L1 antibody comprises the following amino acid sequences: a heavy chain CDR1 region having at least 80% homology to the amino acid sequence set forth in SEQ ID NO: 1 or SEQ ID NO: 4; a heavy chain CDR2 region having at least 80% homology to the amino acid sequence set forth in SEQ ID NO: 2 or SEQ ID NO: 5; a heavy chain CDR3 region having at least 80% homology to the amino acid sequence set forth in SEQ ID NO: 3 or SEQ ID NO: 6; a light chain CDR1 region having at least 80% homology to the amino acid sequence set forth in SEQ ID NO: 7 or SEQ ID NO: 10; a light chain CDR2 region having at least 80% homology to the amino acid sequence set forth in SEQ ID NO: 8 or SEQ ID NO: 11; and a light chain CDR3 region having at least 80% homology to the amino acid sequence set forth in SEQ ID NO: 9 or SEQ ID NO: 12.

**45.** The use according to any one of claims 1-20, or the method according to any one of claims 21-35, or the pharmaceutical combination according to any one of claims 36-43, wherein the anti-PD-L1 antibody comprises the following amino acid sequences: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region

selected from the group consisting of SEQ ID NO: 8 and SEQ ID NO: 11; and a light chain CDR3 region selected from the group consisting of SEQ ID NO: 9 and SEQ ID NO: 12.

**46.** The use according to any one of claims 1-20, or the method according to any one of claims 21-35, or the pharmaceutical combination according to any one of claims 36-43, wherein the anti-PD-L1 antibody comprises: a heavy chain CDR1 region having the amino acid sequence set forth in SEQ ID NO: 1; a heavy chain CDR2 region having the amino acid sequence set forth in SEQ ID NO: 2; a heavy chain CDR3 region having the amino acid sequence set forth in SEQ ID NO: 3; a light chain CDR1 region having the amino acid sequence set forth in SEQ ID NO: 7; a light chain CDR2 region having the amino acid sequence set forth in SEQ ID NO: 8; and a light chain CDR3 region having the amino acid sequence set forth in SEQ ID NO: 9.

**47.** The use according to any one of claims 1-20, or the method according to any one of claims 21-35, or the pharmaceutical combination according to any one of claims 36-43, wherein the anti-PD-L1 antibody comprises the following amino acid sequences: a heavy chain variable region having at least 80% homology to the amino acid sequence set forth in SEQ ID NO: 13 or SEQ ID NO: 14; and a light chain variable region having at least 80% homology to the amino acid sequence set forth in SEQ ID NO: 15 or SEQ ID NO: 16.

**48.** The use according to any one of claims 1-20, or the method according to any one of claims 21-35, or the pharmaceutical combination according to any one of claims 36-43, wherein the anti-PD-L1 antibody comprises: a heavy chain variable region of humanized antibodies selected from the group consisting of hu13C5-hIgG1, hu13C5-hIgG4, hu5G11-hIgG1, and hu5G11-hIgG4; and a light chain variable region of humanized antibodies selected from the group consisting of hu13C5-hIgG1, hu13C5-hIgG4, hu5G11-hIgG1, and hu5G11-hIgG4.

**INTERNATIONAL SEARCH REPORT**

International application No. **PCT/CN2022/110539**

**A. CLASSIFICATION OF SUBJECT MATTER**

A61K 39/395(2006.01)i; A61K 31/4709(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, CNTXT, DWPI, SIPOABS, ENTXT, ENTXTC, CNKI, PubMed, NCBI, EBI-EMBL, ISI Web of Knowledge: 申请人/发明人, PD-L1, CD247, B7-H1, 铂, carboplatin, 依托泊苷, Etoposide, 化疗, 安罗替尼, 安洛替尼, Anlotinib, 拓扑异构酶抑制剂, 小细胞肺癌, SCLC, SEQ ID NOs: 1-16

**C. DOCUMENTS CONSIDERED TO BE RELEVANT**

| Category* | Citation of document, with indication, where appropriate, of the relevant passages | Relevant to claim No. |
|---|---|---|
| X | CHIA TAI TIANQING PHARMACEUTICAL GROUP CO., LTD. "A Study of TQB2450 or Placebo Combined with Anlotinib, Etoposide and Carboplatin Versus Etoposide and Carboplatin in Subjects with Extensive Small Cell Lung Cancer (ETER701)" *NCT04234607,* 22 January 2020 (2020-01-22), Study Description, Arms and Interventions, Criteria | 1-43 |
| Y | CHIA TAI TIANQING PHARMACEUTICAL GROUP CO., LTD. "A Study of TQB2450 or Placebo Combined with Anlotinib, Etoposide and Carboplatin Versus Etoposide and Carboplatin in Subjects with Extensive Small Cell Lung Cancer (ETER701)" *NCT04234607,* 22 January 2020 (2020-01-22), Study Description, Arms and Interventions, Criteria | 44-48 |
| Y | CN 107001463 A (CROWN BIOSCIENCE (TAICANG) INC. et al.) 01 August 2017 (2017-08-01) claims 1-46, and description, embodiment 2 | 44-48 |
| X | HENAN CANCER HOSPITAL. "Alotinib Plus Durvalumab-Platinum-Etoposide in First-line Treatment Extensive Small-cell Lung Cancer" *NCT04660097,* 02 December 2020 (2020-12-02), Study Description, Arms and Interventions | 1-43 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 October 2022** | **04 November 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/110539**

**C. DOCUMENTS CONSIDERED TO BE RELEVANT**

| Category* | Citation of document, with indication, where appropriate, of the relevant passages | Relevant to claim No. |
|---|---|---|
| Y | HENAN CANCER HOSPITAL. "Alotinib Plus Durvalumab-Platinum-Etoposide in First-line Treatment Extensive Small-cell Lung Cancer" *NCT04660097,* 02 December 2020 (2020-12-02), Study Description, Arms and Interventions | 44-48 |
| X | MONTANINOL, A. et al. "Angiogenesis Inhibitors in Small Cell Lung Cancer" *Frontiers in Oncology,* Vol. 11, 28 May 2021 (2021-05-28), Article 655316 | 1-43 |
| Y | MONTANINOL, A. et al. "Angiogenesis Inhibitors in Small Cell Lung Cancer" *Frontiers in Oncology,* Vol. 11, 28 May 2021 (2021-05-28), Article 655316 | 44-48 |
| X | WO 2020015703 A1 (CHIA TAI TIANQING PHARMACEUTICAL GROUP CO., LTD.) 23 January 2020 (2020-01-23) description, p. 2, paragraphs 3-4 and 5, and p. 4, paragraphs 2-8 | 1-43 |
| Y | WO 2020015703 A1 (CHIA TAI TIANQING PHARMACEUTICAL GROUP CO., LTD.) 23 January 2020 (2020-01-23) description, p. 2, paragraphs 3-4 and 5, and p. 4, paragraphs 2-8 | 44-48 |
| X | CN 111617243 A (CHIA TAI TIANQING PHARMACEUTICAL GROUP CO., LTD.) 04 September 2020 (2020-09-04) claims 1-10, and description, paragraphs 22-23 | 1-43 |
| Y | CN 111617243 A (CHIA TAI TIANQING PHARMACEUTICAL GROUP CO., LTD.) 04 September 2020 (2020-09-04) claims 1-10, and description, paragraphs 22-23 | 44-48 |
| A | WO 2020181214 A1 (ADVENCHEN PHARMACEUTICALS, LLC) 10 September 2020 (2020-09-10) entire document | 1-48 |
| A | WO 2020187152 A1 (CHIA TAI TIANQING PHARMACEUTICAL GROUP CO., LTD. et al.) 24 September 2020 (2020-09-24) entire document | 1-48 |
| A | WO 2020233602 A1 (CHIA TAI TIANQING PHARMACEUTICAL GROUP CO., LTD.) 26 November 2020 (2020-11-26) entire document | 1-48 |

Form PCT/ISA/210 (second sheet) (January 2015)

**INTERNATIONAL SEARCH REPORT**

International application No.
**PCT/CN2022/110539**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   [1] The actually submitted sequence table is an XML file of the Standard ST.26.

**INTERNATIONAL SEARCH REPORT**

International application No.
**PCT/CN2022/110539**

**Box No. II Observations where certain claims were found unsearchable (Continuation of item 2 of first sheet)**

This international search report has not been established in respect of certain claims under Article 17(2)(a) for the following reasons:

1. [✓] Claims Nos.: **21-35**, **44-48** (部分)
because they relate to subject matter not required to be searched by this Authority, namely:

[1] Claims 21-35 and 44-48 (in part) relate to a method for treating small-cell lung cancer, and thus do not comply with PCT Rule 39.1(iv). The present report was formed on the basis of amending the subject matter of claims 21-35 and 44-48 (in part) to be a use of a pharmaceutical composition containing an anti-PD-L1 antibody, Anlotinib or a pharmaceutically acceptable salt thereof, and a chemotherapy drug in the preparation of a drug for treating the small-cell lung cancer.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.
PCT/CN2022/110539

| Patent document cited in search report | | | Publication date (day/month/year) | Patent family member(s) | | | Publication date (day/month/year) |
|---|---|---|---|---|---|---|---|
| CN | 107001463 | A | 01 August 2017 | US | 2017204184 | A1 | 20 July 2017 |
| | | | | MX | 2017001597 | A | 17 November 2017 |
| | | | | BR | 112017002234 | A2 | 24 July 2018 |
| | | | | AU | 2015301126 | A1 | 23 February 2017 |
| | | | | CN | 110964109 | A | 07 April 2020 |
| | | | | KR | 20170039706 | A | 11 April 2017 |
| | | | | CA | 2956399 | A1 | 11 February 2016 |
| | | | | SG | 11201700687T | A | 27 February 2017 |
| | | | | AU | 2021203593 | A1 | 01 July 2021 |
| | | | | WO | 2016022630 | A1 | 11 February 2016 |
| | | | | RU | 2017106804 | A | 06 September 2018 |
| | | | | JP | 2017523786 | A | 24 August 2017 |
| | | | | US | 2020031935 | A1 | 30 January 2020 |
| | | | | IL | 250415 | D0 | 30 March 2017 |
| | | | | US | 2021371531 | A1 | 02 December 2021 |
| | | | | SG | 10201901057U | A | 28 March 2019 |
| | | | | JP | 2020141672 | A | 10 September 2020 |
| | | | | ZA | 201700785 | B | 28 July 2021 |
| | | | | EP | 3177649 | A1 | 14 June 2017 |
| | | | | CN | 110964108 | A | 07 April 2020 |
| WO | 2020015703 | A1 | 23 January 2020 | CN | 112566661 | A | 26 March 2021 |
| | | | | EP | 3842070 | A1 | 30 June 2021 |
| | | | | JP | 2022512526 | A | 07 February 2022 |
| | | | | CA | 3106055 | A1 | 23 January 2020 |
| | | | | AU | 2019305857 | A1 | 18 February 2021 |
| | | | | US | 2022160700 | A1 | 26 May 2022 |
| | | | | KR | 20210034613 | A | 30 March 2021 |
| CN | 111617243 | A | 04 September 2020 | | None | | |
| WO | 2020181214 | A1 | 10 September 2020 | CA | 3132670 | A1 | 10 September 2020 |
| | | | | US | 2022054475 | A1 | 24 February 2022 |
| | | | | KR | 20210151806 | A | 14 December 2021 |
| | | | | IL | 286183 | A | 31 October 2021 |
| | | | | EP | 3934645 | A1 | 12 January 2022 |
| | | | | AU | 2020231236 | A1 | 04 November 2021 |
| | | | | CN | 113518621 | A | 19 October 2021 |
| | | | | JP | 2022524761 | A | 10 May 2022 |
| WO | 2020187152 | A1 | 24 September 2020 | EP | 3939610 | A1 | 19 January 2022 |
| | | | | CA | 3133141 | A1 | 24 September 2020 |
| | | | | CN | 113613674 | A | 05 November 2021 |
| | | | | US | 2022175759 | A1 | 09 June 2022 |
| | | | | AU | 2020242144 | A1 | 04 November 2021 |
| WO | 2020233602 | A1 | 26 November 2020 | AU | 2020279289 | A1 | 06 January 2022 |
| | | | | JP | 2022533211 | A | 21 July 2022 |
| | | | | EP | 3973964 | A1 | 30 March 2022 |
| | | | | US | 2022193066 | A1 | 23 June 2022 |
| | | | | CN | 113811298 | A | 17 December 2021 |
| | | | | KR | 20220011647 | A | 28 January 2022 |
| | | | | CA | 3141167 | A1 | 26 November 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016022630 A
- CN 107001463 A